# EUROPEAN PATENT APPLICATION

(11) **EP 1 306 433 A1**
(43) Date of publication of application: **02.05.2003**
(21) Application number: 01955561.4
(22) Date of filing: 02.08.2001
(51) Int. Cl.: C12N 15/11, C12Q 1/68

(54) **MARKER FOR DETECTING PLANT GENOME POLYMORPHISM WITH THE USE OF TRANSPOSABLE ELEMENT AND METHOD OF CONSTRUCTING THE SAME**

(30) Priority: 02.08.2000 JP 2000234577
(71) Applicant: Japan Tobacco Inc., Tokyo 105-8422 (JP); Syngenta Limited, Guildford Surrey GU2 7YH (GB)
(72) Inventor: KOMORI, Toshiyuki, Iwata-gun, Shizuoka 438-0802 (JP); NITTA, Naoto, Iwata-gun, Shizuoka 438-0802 (JP)
(74) Representative: Reinhard - Skuhra - Weise & Partner GbR
(86) International application number: JP0106661
(87) International publication number: WO02012484

(57) **Abstract**

The present invention aims to provide a marker for detecting a polymorphism in a plant genome using a transposable element and a method for producing thereof. The method for producing a marker for detecting a polymorphism in a plant genome is characterized in that it comprises preparing primer for nucleic acid amplification using a base sequence of transposable element and/or adjacent region thereto in plant genome.

## Description

### FIELD OF THE INVENTION

The present invention relates to markers for detecting plant genomic polymorphisms and a method for producing thereof.

The present application claims priority based on Japanese Patent Application No. 2000-234557 filed on August 2, 2000, the whole disclosure of which is incorporated herein.

### BACKGROUND ART

### Genetic polymorphism

The presence of diversity in a certain trait or morphology between normal individuals within the same species is called "polymorphism". Especially, polymorphisms resulting from genetic differences are called "genetic polymorphisms". Evidence has shown that natural populations carry many genetic variations such as polymorphisms involved in the structure or morphology of a chromosome that can be cytogenetically detected and polymorphisms involved in the allele at a specific locus (Dictionary of Biochemistry, 3rd Edition, p. 835, published on October 8, 1998 by Tokyo Kagaku Dozin).

A typical example of genetic polymorphism is shown in elytral macula in Harmonia axyridis including at least four alleles. Chromosomal polymorphisms characterized by inversion or other chromosomal variations carried as polymorphisms in a species are well studied in various species of Drosophila. Among chromosomal polymorphisms, "DNA polymorphism" means a variation found at one amino acid site or nucleotide site (Iwanami's Dictionary of Biology, 4th Edition, p. 81, published on March 21, 1996 by Iwanami Shoten Publishers).

Many DNA polymorphisms have also been detected in plant genomes (e.g. monocotyledons such as rice, wheat and maize; dicotyledons such as Arabidopsis and tobacco; woody plants such as pearwood and Japanese cedar). For example, a linkage map for rice has been produced with a marker based on DNA polymorphisms and used for genetic analysis or breeding selection (Harushima et al., Genetics 148: pp. 479-494, January 1998).

Conventional approaches for detecting DNA polymorphisms include those such as restriction fragment length polymorphism (RFLP) analysis; direct method by sequencing; or a method involving cleavage of genomic DNA with an 8-base recognizing restriction enzyme followed by radioactive end-labeling, cleavage with 6-base and 4-base recognizing restriction enzymes and development by two-dimensional electrophoresis (RLGS: Restriction Landmark Genome Scanning). AFLP (amplified fragment length polymorphism: P. Vos et al., Nucleic Acids Res., Vol. 23, pp. 4407-4414 (1995)) analysis was also developed for amplifying/detecting RFLP via polymerase chain reaction (PCR). However, each method has problems and an alternative method capable of more efficiently detecting polymorphisms is needed.

More specifically, conventional approaches rely on detecting RFLPs via PCR amplification (conversion of RFLP markers into PCR markers) or detecting polymorphisms in microsatellites via PCR amplification (microsatellite markers) as illustrated below, for example.

### (1) Conversion of RFLP markers into PCR markers

A. PCR markers based on polymorphisms in genomic regions corresponding to RFLP probes (D.E. Harry, B.Temesgen, D.B. Neale; Codominant PCR-based markers for Pinus taeda developed from mapped cDNA clones, Theor. Appl. Genet. (1998) 97: pp. 327-336).

After performing genomic PCR using primers designed for an RFLP marker probe sequence ("RFLP" is a polymorphism observed by Southern analysis using a DNA fragment as a probe. The nucleotide sequence of the DNA fragment used as a probe is called "RFLP marker probe sequence"), a PCR marker can be obtained by either of the following two procedures. A first procedure involves treating the products with a series of restriction enzymes to search for a restriction enzyme causing a fragment length polymorphism, and a second procedure involves searching for a polymorphism by intervarietal comparison of the nucleotide sequences of the products and obtaining a PCR marker based on the polymorphism.

However, this method involves the problem that the frequency of intervarietal polymorphisms is so low that when PCR products of about 1000 bp were compared, not only could RFLP not be obtained but also, in many cases, no intervarietal polymorphism was found even on nucleotide sequence level. Comparison over longer regions may be possible, but more work is required.

### B. PCR markers based on identification of RFLP-causing sites

A PCR marker can be obtained by identifying an RFLP-causing site (a restriction enzyme recognition site carried by only one of two varieties compared) present in or near (normally within several kbs) an RFLP marker probe sequence.

However, this method involves the problem that it is highly time and work intensive because genomic clones containing a genomic region corresponding to the RFLP marker probe and its surrounding regions must be isolated/analyzed in order to identify an RFLP-causing site. Specifically, a series of experiments are required to be conducted including screening of genomic libraries (hybridization), amplification and isolation of positive clones, production of restriction maps of the isolated clones, identification of restriction sites causing polymorphisms, designing of suitable primers and identification of polymorphisms, which require about one month even if all goes well.

### (2) Microsatellite markers

Microsatellites are repeated sequences of about 2 to 4 nucleotides such as (CA)n that are present in great numbers in genomes. If a intervarietal polymorphism occurs in repetition number, a polymorphism can be observed in PCR product length by PCR using primers designed in adjacent regions, whereby the DNA polymorphism can be detected. Markers for detecting polymorphisms using microsatellites are called microsatellite markers (O. Parnaud, X. Chen, S.R. McCouch, Development of microsatellite markers and characterization of simple sequence length polymorphism (SSLP) in rice (Oryza sativa L.), Mol. Gen. Genet. (1996) 252: pp. 597-607).

However, this method involves the problem that randomly selected microsatellites often fail to show polymorphism. Moreover, polymorphisms can be clearly observed with many microsatellite markers only after analysis on acrylamide gels, which is much more complex than normal analysis on agarose gel.

As described above, various approaches for detecting DNA polymorphisms have been studied, but markers and methods for more efficiently detecting polymorphisms are needed.

### Transposable elements

"Transposable element" is synonymous with "transposable genetic element", "mobile genetic element" or "mobile element", and means a DNA unit having a defined structure that can transpose from one site to another site on DNA. It is normally flanked at each end by an inverted sequence of about 10-50 base pairs and it normally contains an internal sequence encoding a transposase, i.e. an enzyme recognizing this terminal inverted sequence to control transposition reaction. It inactivates a gene or alters a gene structure by simple insertion or excision, but also induces deletion, inversion, duplication and replicon fusion by transposition reaction to mediate genomic rearrangements.

The presence of transposable elements was shown in a genetic system of maize by B. McClintock. Examples in prokaryotes include insertion sequences (IS), transposons (Tn) carrying genes for resistance to antibiotics or the like and lysogenic phages such as Mu. Well-known examples in eukaryotes are Ac and Spm in maize, Tam in Antirrhinum majus, P element in Drosophila and Tc1 in nematodes.

Recombination reaction by transposition is characterized by duplication of a sequence of several base pairs at a target site as also observed in the event where the cDNA generated by reverse transcription from the RNA of retroviruses/retro(trans)posons present in eukaryotes is integrated by the action of an integration enzyme, an integrase (IN protein). Therefore, these elements are also included in transposable elements in a broad sense.
Genetic engineering techniques based on the transposability of transposable elements such as transposon labeling have been applied to various biosystems (Iwanami's Dictionary of Biology, Fourth Edition, pp. 966-967, published March 21, 1996 by Iwanami Shoten Publishers).

### (1) Transposons

Transposable elements in general or especially those carrying marker genes such as drug resistance genes found in chromosomes of prokaryotes or plasmids are called "transposons" (Iwanami's Dictionary of Biology, 4th Edition, p. 1011, supra.).

Transposons are classified by the origin or the type of the marker gene as Tn1, Tn2, Tn3... They are classified by their structural feature into those flanked at each end by an insertion sequence or a related sequence and those flanked by only an inverted repeat sequence. Examples of the former include Tn5 and Tn9, and examples of the latter include Tn3. Tn5 has a length of about 5.8 kilobase pairs (bp) containing insertion sequences (IS50) of 1533 bp oriented in opposite directions at both ends and an internal gene for resistance to kanamycin. Tn9 has insertion sequences (IS1) oriented in the same direction at both ends and an internal gene for resistance to chloramphenicol. These Tns are supplied with a transposase, an enzyme necessary for transposition from their own insertion sequences. Tn3 differs from these transposons in that it is flanked by an inverted repeat sequence of 38 bp at each end and has an internal sequence encoding its own transposase and an ampicillin resistance gene.

It is generally considered that once a transposable element is inserted onto DNA, a nucleotide sequence targeted by the insertion is duplicated outside the transposable element with the number of nucleotides duplicated (generally, about 3-12 base pairs) being defined by the type of transposase. Such duplication of the target site is characteristic of the integration of transposable elements.

The term "transposons" sometimes broadly means transposable elements in general including retrotransposons in eukaryotes described below.

### (2) Retroposons

When information (DNA) at a site in a genome is transcribed into RNA and then transferred to complementary DNA by the action of a reverse transferase and reinserted into another site in the genome, such DNA is collectively called "retroposon". In contrast, DNA transposing as DNA without any intermediate is called "transposon" in a narrow sense. Most interspersed repeat sequences are retroposons (Iwanami's Dictionary of Biology, 4th Edition, p. 1500, supra.).

Retroposons are mainly divided into the following two families.
(1) Viral family: include retroviruses and retroposons encoding a reverse transferase by themselves such as Copia elements in Drosophila, Ty element in yeast and L1 element in human. Retroposons in the viral family except for retroviruses are collectively called retrotransposons and their transposition events are called retrotranspositions.
(2) Non-viral family: include pseudogenes generated by reverse transcription from mRNA transcribed from a gene encoding a protein by RNA polymerase II, pseudogenes of intranuclear low molecular RNA and interspersed short repeat sequences transcribed by RNA polymerase III as collectively called SINEs. SINEs include the Alu family present in human genomes and many other known examples derived from tRNA (P.L. Deininger (1989), SINEs: Short interspersed repeated DNA elements in higher eukaryotes. In: Mobile DNA (eds.: D.E. Berg and M.M. Howe), pp .619-636 American Society for Microbiology, Washington, D.C.). In a narrow sense, only retroposons of the non-viral family are sometimes called retroposons. Retroposons are often thought to be parasitic elements with no function, but rare cases where retroposons acquired a function or a gene was newly generated by retroposition are known.

The presence of many transposable elements in plants has also been reported. Plant transposable elements are here grouped into class I and class II according to the classification of Ohtsubo (Cell Technology, Supplementary Volume, Plant Cell Technology Series 5, Plant Genome Science, pp. 102-113, published on October 1, 1996 by Shujunsha Co. Ltd.). Class I consists of retroposons transposing via an RNA intermediate including retrotransposons, LINES and SINEs. Class II consists of transposons transposing as DNA without forming any intermediate including Ac/Ds, En/Spm and Mu. Novel transposons recently discovered from plant genomes called MITEs (Miniature Inverted Repeat Transposable Elements) are also classified as class II for convenience, but their transposition mechanism has not been explained yet.

MITEs are structurally characterized by the fact that they are relatively short (at most about 400 bp), do not encode any gene, are flanked at each end by an inversed repeat, may have the secondary structure of DNA, and that each MITE element has specific target sequence (S.R. Wessler et al., Current Opinion in Genetics & Development 1995, Vol. 5, pp. 814-821). Each MITE element is presumed to occur in great numbers throughout plant genomes. For example, Tnr1 belonging to Stowaway occurs in about 3500 copies in rice genomes (T. Tenzen et al., Mol Gen Genet (1994) Vol. 245: pp. 441-448). B2 and Hbr elements belonging to Tourist found in maize are each estimated at 10,000 copies or more (S.R. Wessler et al., Current Opinion in Genetics & Development 1995, Vol. 5, pp. 814-821).

Plant transposable elements are found in rice, maize, tobacco, etc. Especially in rice, retrotransposons such as Tos (H. Hirochika et al., Proc. Natl. Acad. Sci. USA (1996) Vol. 93, pp. 7783-7788), RIRE1 (K. Noma et al., Genes Genet. Syst. (1997) Vol. 72 pp.131-140) and RIRE2 (H. Ohtsubo et al., Genes Genet. Syst. (1999) Vol. 74 pp. 83-91) and SINEs such as p-SINE1 (K. Mochizuki et al., Jpn. J. Genet. (1992) 57, pp. 155-166) are known. In class II, Tnr3 (R. Motohashi et al., Mol Gen Genet (1996) Vol. 250, pp. 148-152) belonging to En/Spm and MITEs are known. Recently, W.-Y. Song et al. (Mol Gen Genet (1998) Vol. 258: 449-456) discovered new elements belonging to class II such as Krispie, Snap, Crackle, Pop and Truncator.

Known MITEs include Gaijin, Castaway, Ditto, Wanderer and Explorer (T. Bureau et al., Proc. Natl. Acad. Sci. USA, Vol. 93, pp.8524-8529, August 1996), Tourist (T.E. Bureau et al., Proc. Natl. Acad. Sci. USA (1994) Vol. 91, pp. 1411-1415), Stowaway (T.E. Bureau et al., The Plant Cell (1994) Vol. 6, pp. 907-916), Amy/LTP (N. Huang et al., Gene (1992) Vol. 111, pp. 223-228, F. Vignols et al., Gene (1994) Vol. 142, pp. 265-270, T. Bureau et al., Proc. Natl. Acad. Sci. USA, Vol. 93, pp. 8524-8529, August 1996), Tnr2 (K. Mochizuki et al., Jpn. J. Genet. (1993) Vol. 68, pp. 205-217), etc.

Transposable elements of the present invention are not limited to known transposable elements listed above.

The paper of K. Mochizuki et al., Jpn. J. Genet. (1992) 57, pp. 155-166 mentioned above suggests that mutational base substitution are more likely to occur in repeat sequences than in unique sequences in p-SINE1. However, they neither specifically discuss the relationship between transposable elements and polymorphisms nor refer to the applicability of transposable elements as markers for detecting polymorphisms.

### DISCLOSURE OF THE INVENTION

An object of the present invention is to provide a method for producing a marker for detecting a polymorphism in a plant genome. The method of the present invention is characterized in that it comprises preparing primer for nucleic acid amplification using a base sequence of transposable element and/or adjacent region thereto in plant genome.

In one embodiment, the method of the present invention comprises:
i) preparing a pair of primers based on a base sequence of transposable element and/or adjacent region thereto present in genome of a target plant species to amplify said transposable element, or both the transposable element and the adjacent region;
ii) performing nucleic acid amplification reaction using genome DNA of the target plant species as a template; and
iii) producing a marker for detecting polymorphism in a plant genome based on polymorphism found in said nucleic acid amplification product.

Another object of the present invention is to provide a marker for detecting a polymorphism in a plant genome produced by the method of the present invention.

### BRIEF DESCRIPTION OF THE DRAWINGS

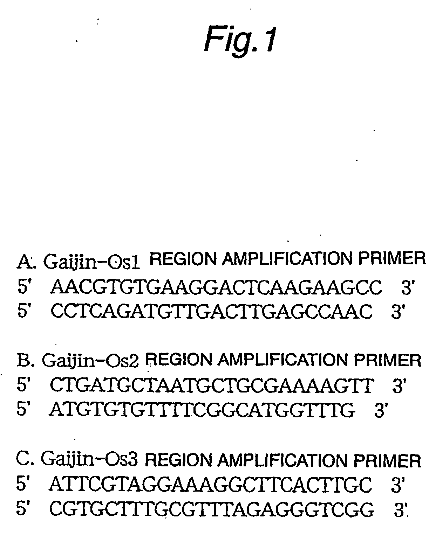

FIG. 1 shows the sequences of primers for amplifying Gaijin regions.

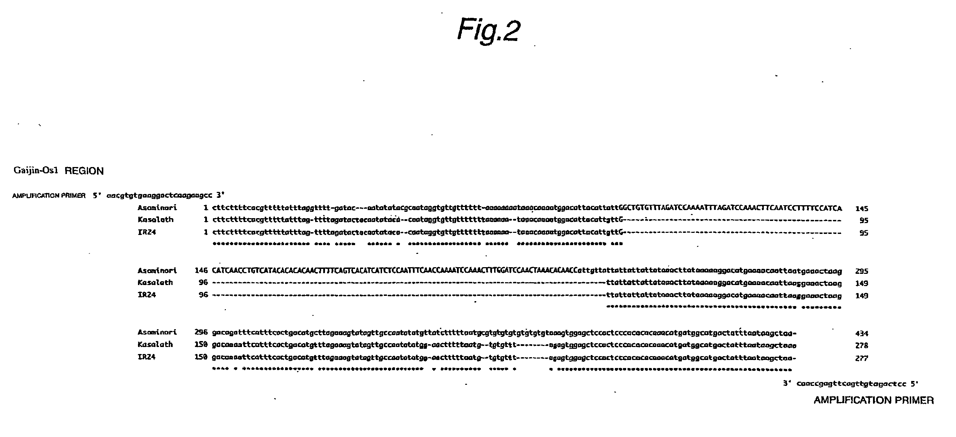

FIG. 2 shows a intervarietal comparison of the nucleotide sequences of Gaijin 1 region. Capital and lowercase letters indicate Gaijin-Os1 sequence and adjacent sequences thereto, respectively. Blanks indicate polymorphic sites distinct from the sequence of Asominori.

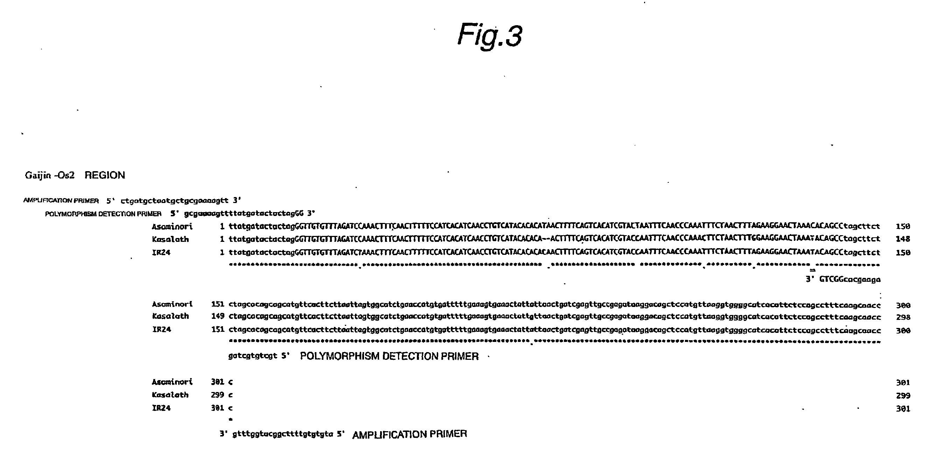

FIG. 3 shows a intervarietal comparison of the nucleotide sequences of Gaijin 2 region. Capital and lowercase letters indicate Gaijin-Os2 sequence and adjacent sequences thereto, respectively. Blanks indicate polymorphic sites distinct from the sequence of Asominori. The polymorphism used for producing a PCR marker is indicated by double underlining.

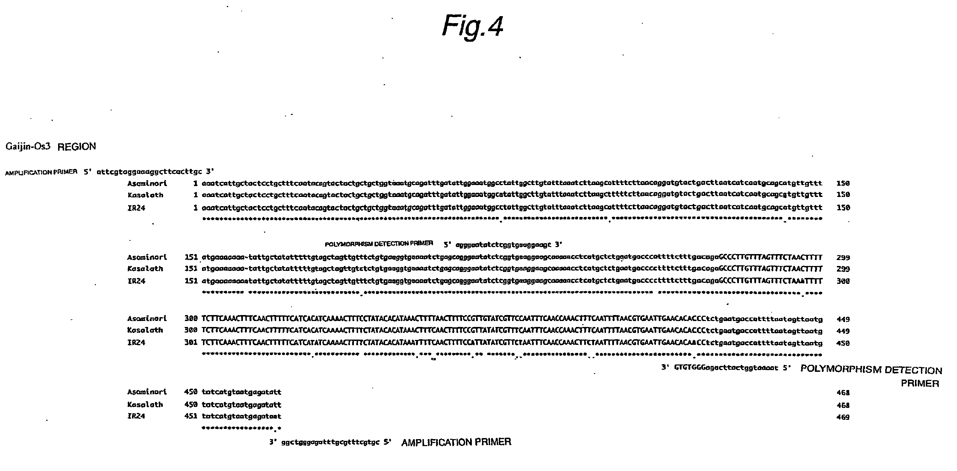

FIG. 4 shows a intervarietal comparison of the nucleotide sequences of Gaijin 3 region. Capital and lowercase letters indicate Gaijin-Os3 sequence and adjacent sequences thereto, respectively. Blanks indicate polymorphic sites distinct from the sequence of Asominori. The polymorphism used for producing a PCR marker is indicated by double underlining.

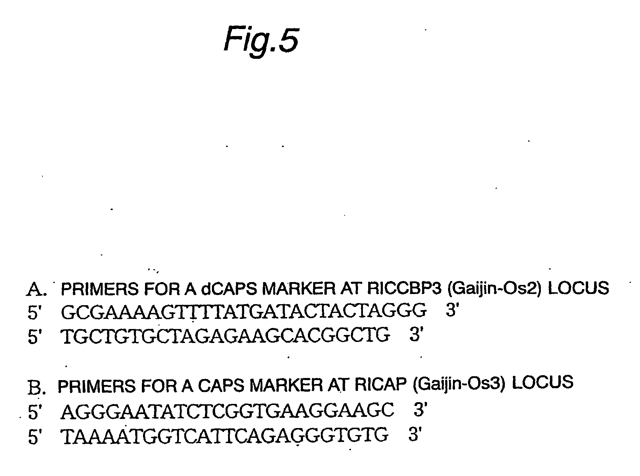

FIG. 5 shows the sequences of primers as (PCR) markers for detecting polymorphisms in Gaijin regions.

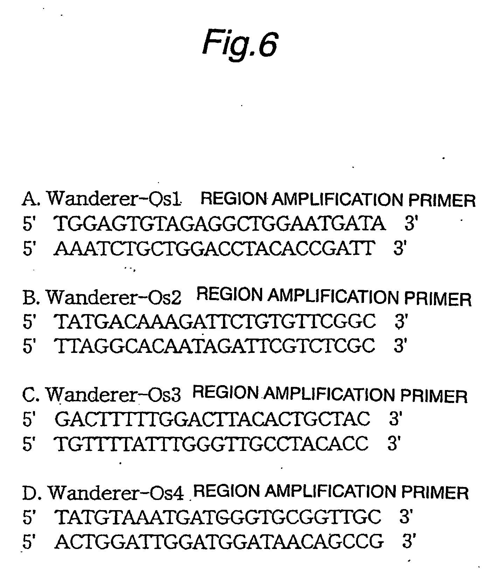

FIG. 6 shows the sequences of primers for amplifying Wanderer regions.

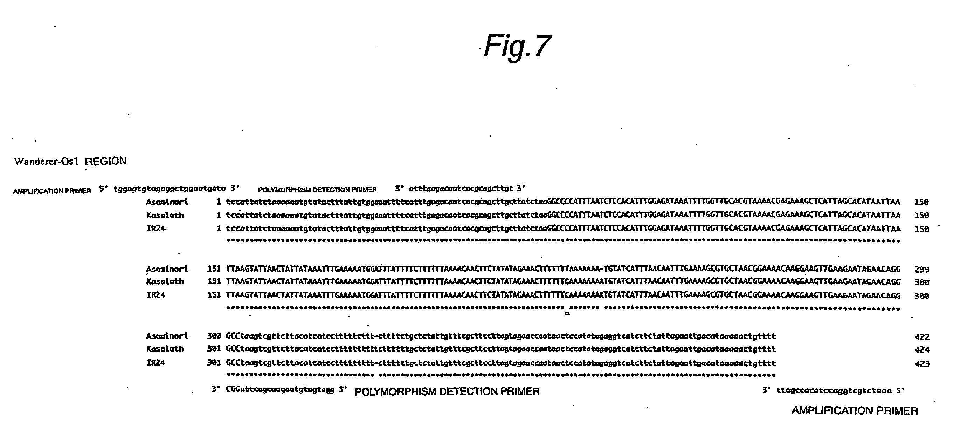

FIG. 7 shows a intervarietal comparison of the nucleotide sequences of Wanderer 1 region. Capital and lowercase letters indicate Wanderer-Os1 sequence and adjacent sequences thereto, respectively. Blanks indicate polymorphic sites distinct from the sequence of Asominori. The polymorphism used for producing a PCR marker is indicated by double underlining.

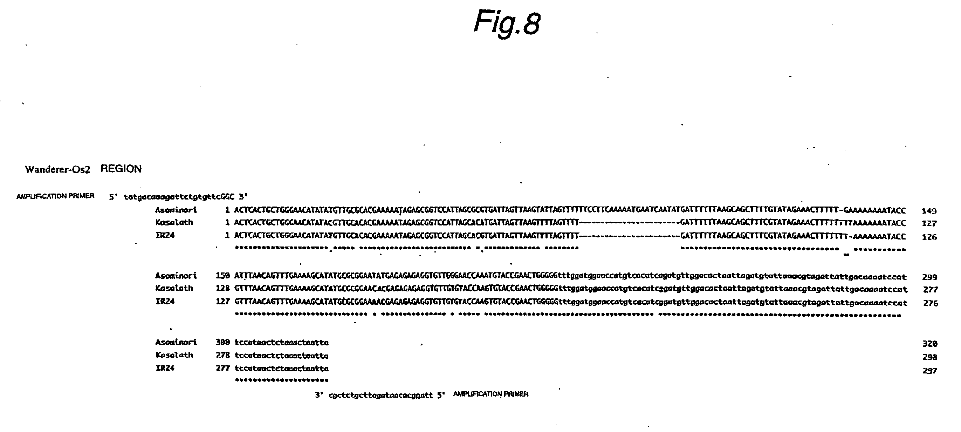

FIG. 8 shows a intervarietal comparison of the nucleotide sequences of Wanderer 2 region. Capital and lowercase letters indicate Wanderer-Os2 sequence and adjacent sequences thereto, respectively. Blanks indicate polymorphic sites distinct from the sequence of Asominori.

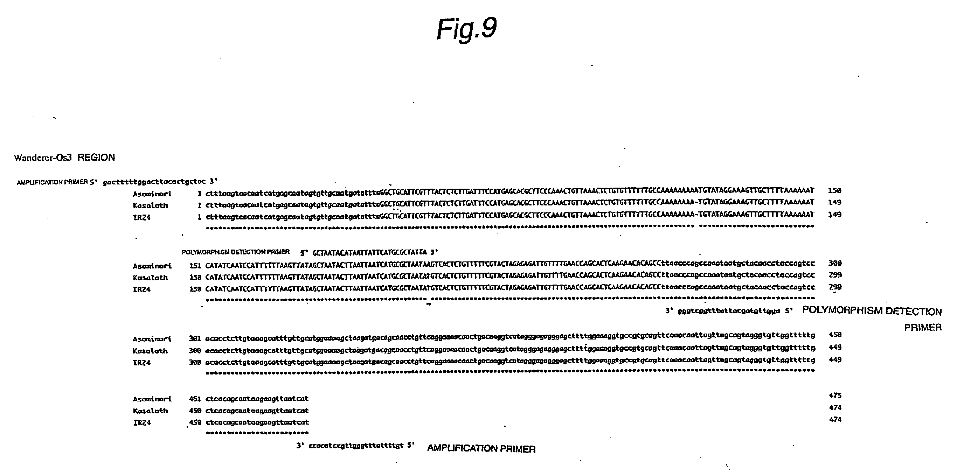

FIG. 9 shows a intervarietal comparison of the nucleotide sequences of Wanderer 3 region. Capital and lowercase letters indicate Wanderer-Os3 sequence and adjacent sequences thereto, respectively. Blanks indicate polymorphic sites distinct from the sequence of Asominori. The polymorphism used for producing a PCR marker is indicated by double underlining.

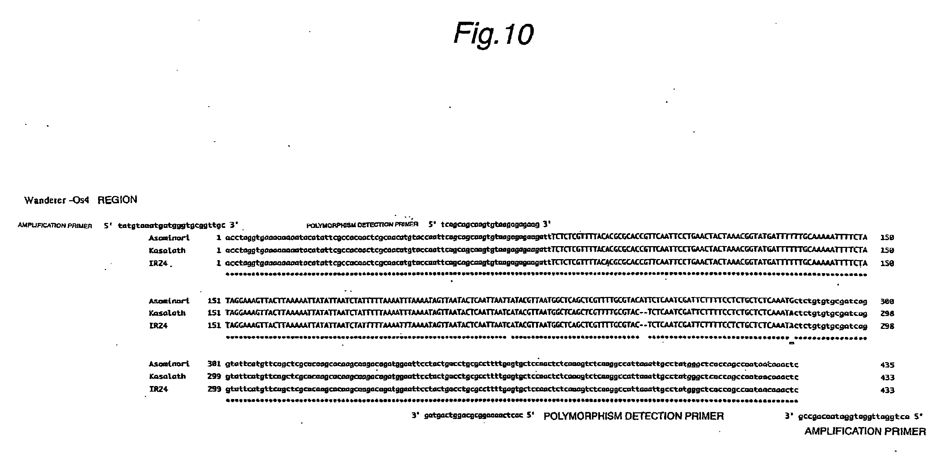

FIG. 10 shows a intervarietal comparison of the nucleotide sequences of Wanderer 4 region. Capital and lowercase letters indicate Wanderer-Os4 sequence and adjacent sequences thereto, respectively. Blanks indicate polymorphic sites distinct from the sequence of Asominori. The polymorphism used for producing a PCR marker is indicated by double underlining.

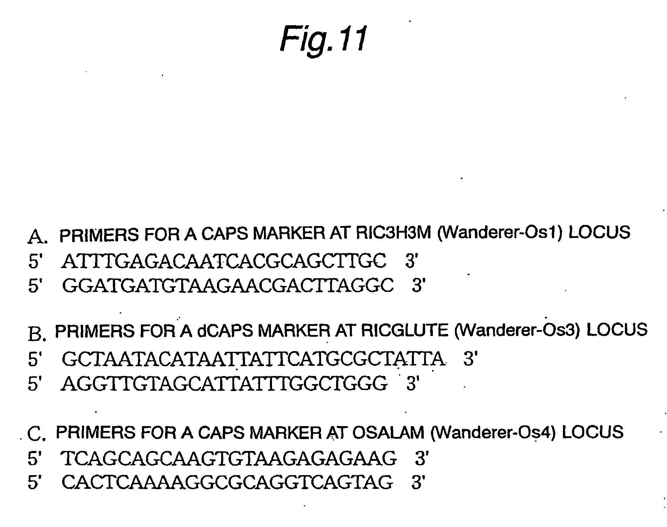

FIG. 11 shows the sequences of primers as (PCR) markers for detecting polymorphisms in Wanderer regions.

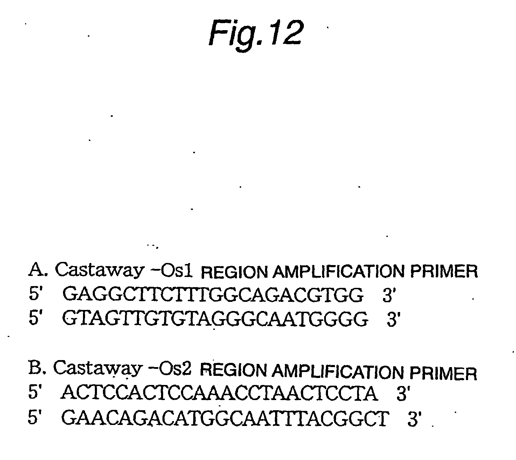

FIG. 12 shows the sequences of primers for amplifying Castaway regions.

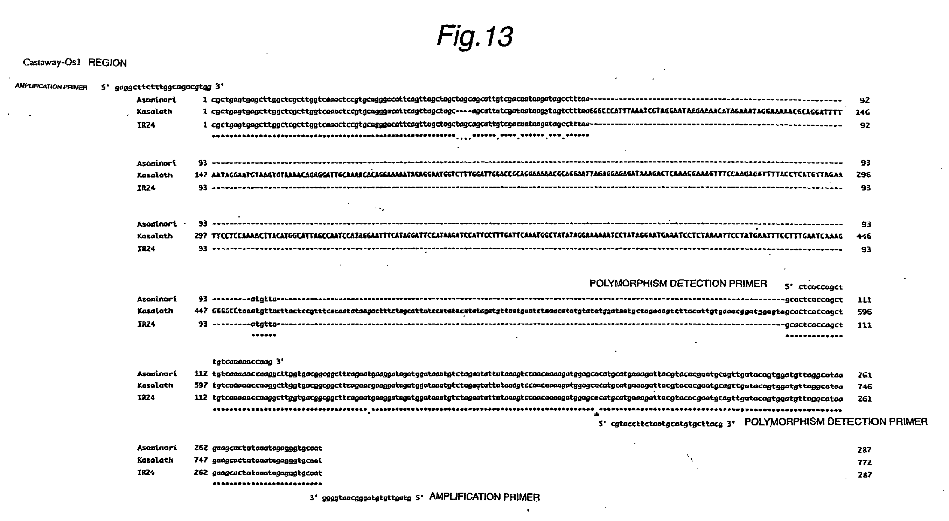

FIG. 13 shows a intervarietal comparison of the nucleotide sequences of Castaway 1 region. Capital and lowercase letters indicate Castaway-Os1 sequence and adjacent sequences thereto, respectively. Blanks indicate polymorphic sites distinct from the sequence of Asominori. The polymorphism used for producing a PCR marker is indicated by double underlining.

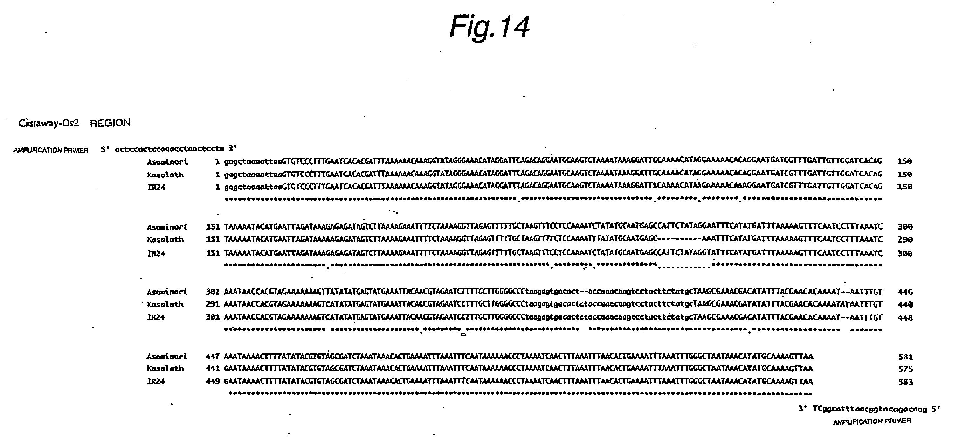

FIG. 14 shows a intervarietal comparison of the nucleotide sequences of Castaway 2 region. Capital and lowercase letters indicate Castaway-Os2 sequence and adjacent sequences thereto, respectively. Blanks indicate polymorphic sites distinct from the sequence of Asominori. The polymorphism used for producing a PCR marker is indicated by double underlining. Later capital letters indicate another transposable element Explorer-Os2.

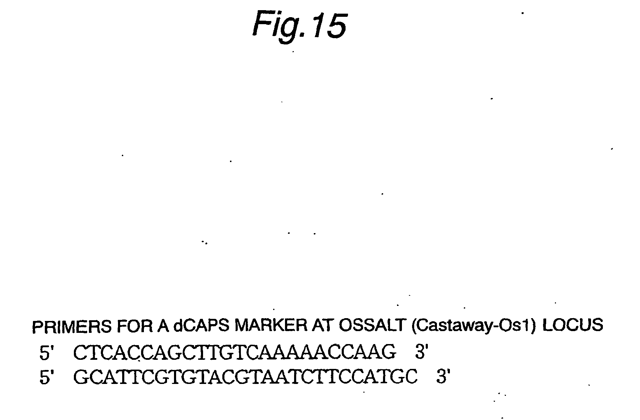

FIG. 15 shows the sequences of primers as (PCR) markers for detecting polymorphisms in a Castaway region.

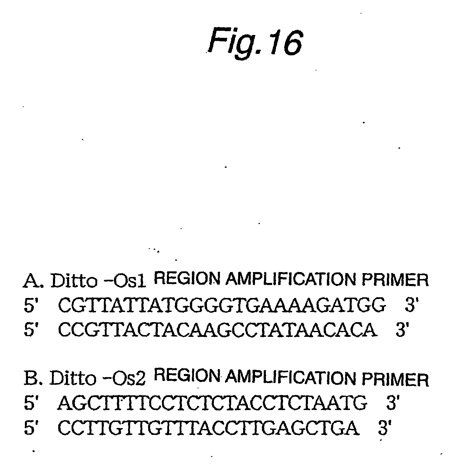

FIG. 16 shows the sequences of primers for amplifying Diito regions.

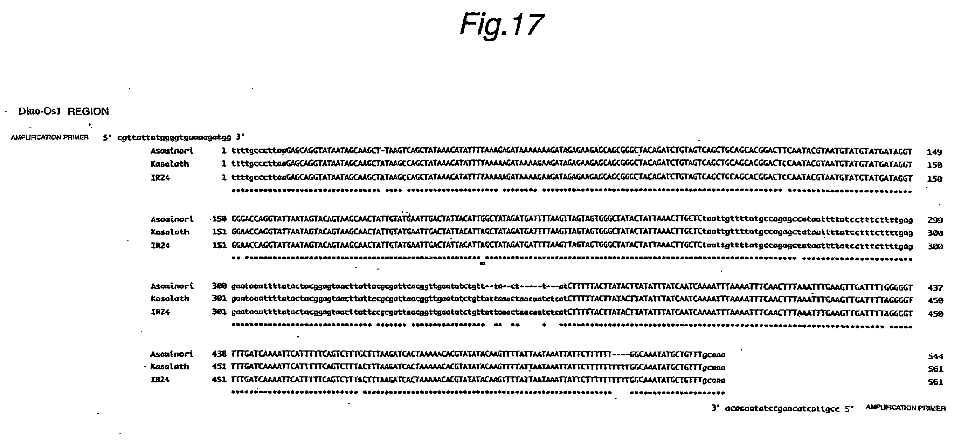

FIG. 17 shows a intervarietal comparison of the nucleotide sequences of Ditto 1 region. Capital and lowercase letters indicate Ditto-Os1 sequence and adjacent sequences thereto, respectively. Blanks indicate polymorphic sites distinct from the sequence of Asominori. The polymorphism used for producing a PCR marker is indicated by double underlining. Capital letters in the latter portion indicate another transposable element Explorer-Os2.

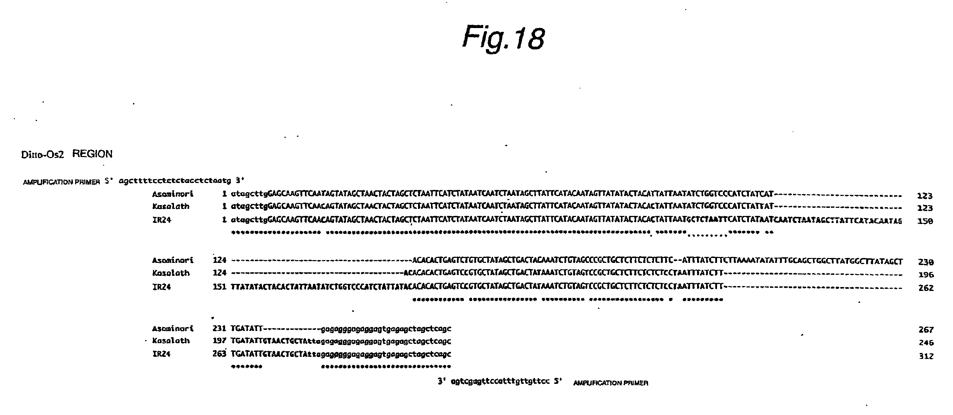

FIG. 18 shows a intervarietal comparison of the nucleotide sequences of Ditto 2 region. Capital and lowercase letters indicate Ditto-Os2 sequence and adjacent sequences thereto, respectively. Blanks indicate polymorphic sites distinct from the sequence of Asominori.

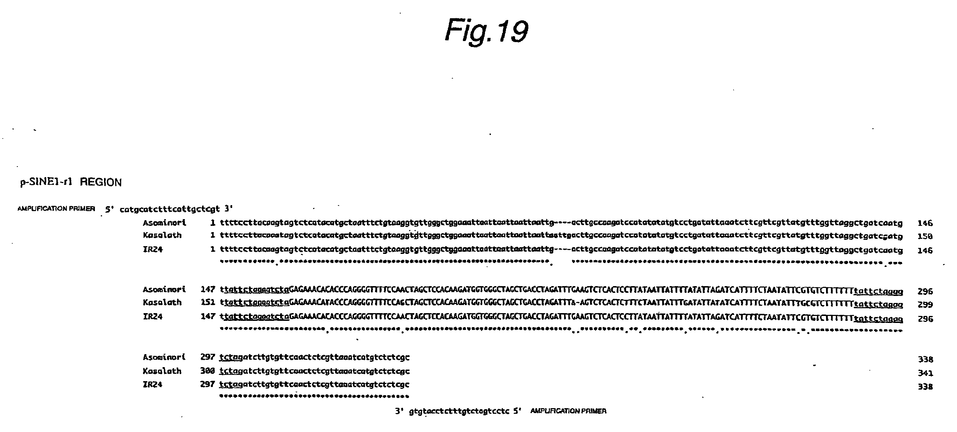

FIG. 19 shows a intervarietal comparison of the nucleotide sequences of p-SINE-r1 region. Capital and lowercase letters indicate p-SINE-r1 sequence and adjacent sequences thereto, respectively. Blanks indicate polymorphic sites distinct from the sequence of Asominori. Single underlining indicates forward repeat sequences.

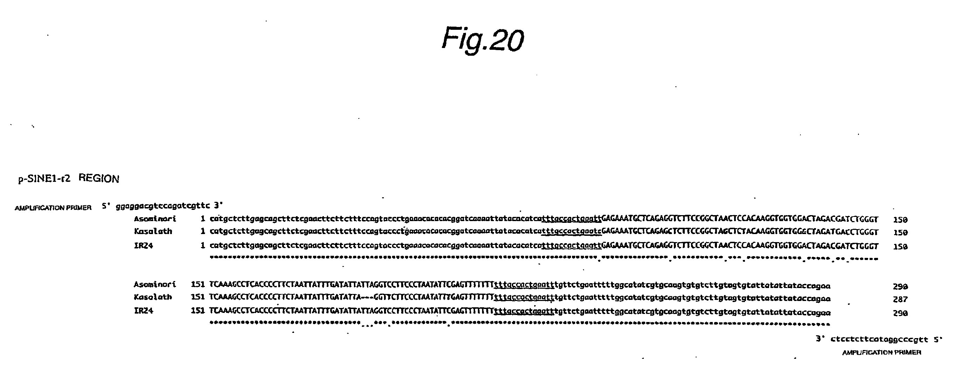

FIG. 20 shows a intervarietal comparison of the nucleotides sequence of p-SINE-r2 region. Capital and lowercase letters indicate p-SINE-r2 sequence and adjacent sequences thereto, respectively. Blanks indicate polymorphic sites distinct from the sequence of Asominori. Single underlining indicates forward repeat sequences.

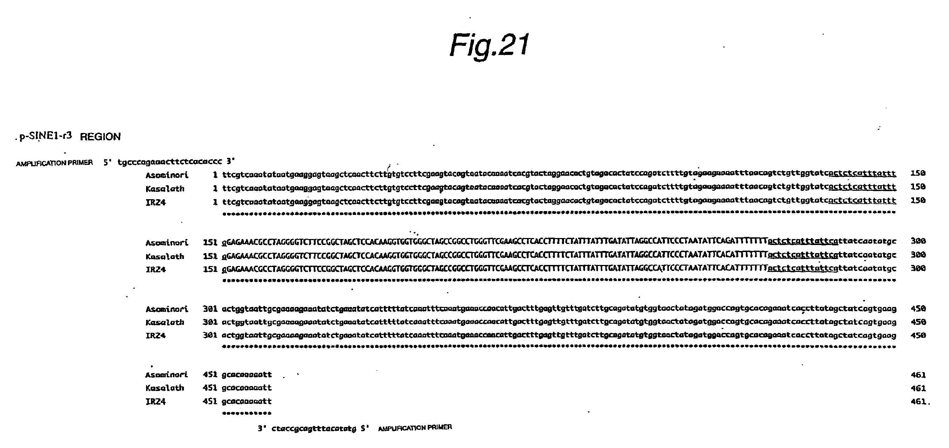

FIG. 21 shows a intervarietal comparison of the nucleotide sequences of p-SINE-r3 region. Capital and lowercase letters indicate p-SINE-r3 sequence and adjacent sequences thereto, respectively. Single underlining indicates forward repeat sequences.

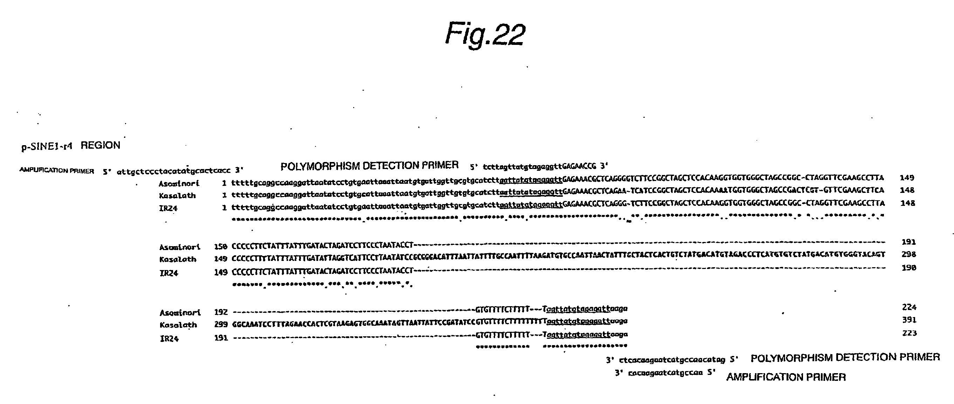

FIG. 22 shows a intervarietal comparison of the nucleotide sequences of p-SINE-r4 region. Capital and lowercase letters indicate p-SINE-r4 sequence and adjacent sequences thereto, respectively. Blanks indicate polymorphic sites distinct from the sequence of Asominori. Single underlining indicates forward repeat sequences. The polymorphism used for producing a PCR marker is indicated with double underlining.

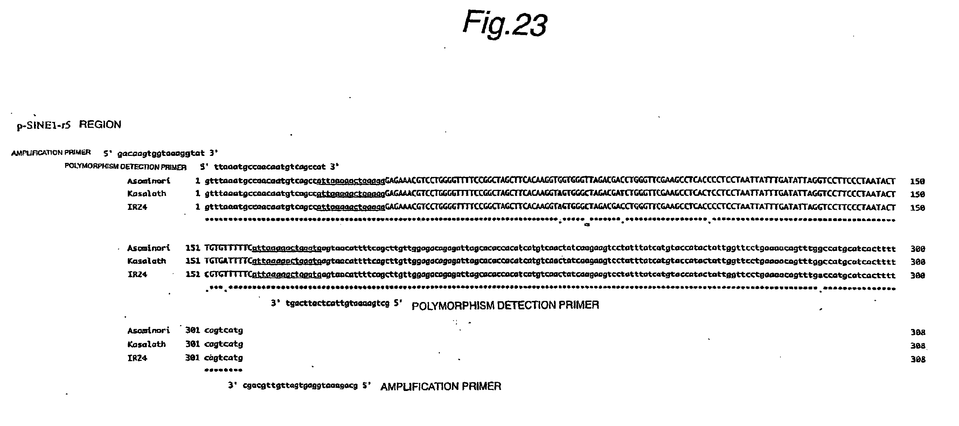

FIG. 23 shows a intervarietal comparison of the nucleotide sequences of p-SINE-r5 region. Capital and lowercase letters indicate p-SINE-r5 sequence and adjacent sequences thereto, respectively. Blanks indicate polymorphic sites distinct from the sequence of Asominori. Single underlining indicates forward repeat sequences. The polymorphism used for producing a PCR marker is indicated by double underlining.

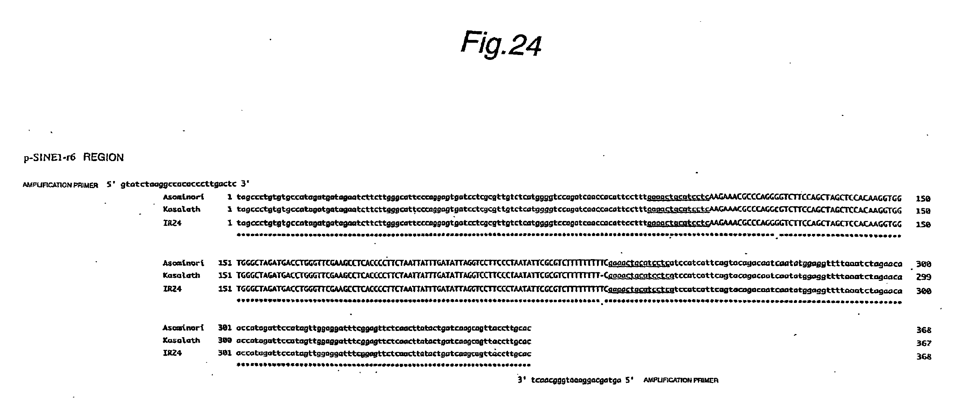

FIG. 24 shows a intervarietal comparison of the nucleotide sequences of p-SINE-r6 region. Capital and lowercase letters indicate p-SINE-r6 sequence and adjacent sequences thereto, respectively. Blanks indicate polymorphic sites distinct from the sequence of Asominori. Single underlining indicates forward repeat sequences.

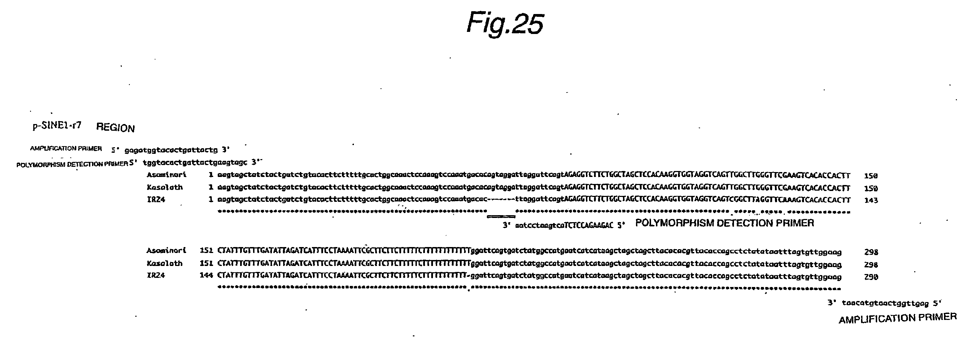

FIG. 25 shows a intervarietal comparison of the nucleotide sequences of p-SINE-r7 region. Capital and lowercase letters indicate p-SINE-r7 sequence and adjacent sequences thereto, respectively. Blanks indicate polymorphic sites distinct from the sequence of Asominori. Single underlining indicates forward repeat sequences. The polymorphism used for producing a PCR marker is indicated by double underlining.

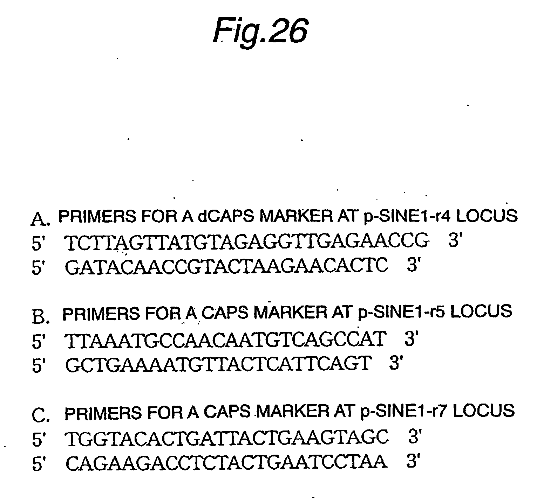

FIG. 26 shows the sequences of primers as (PCR) markers for detecting polymorphisms in p-SINE regions.

### DETAILED DESCRIPTION OF THE INVENTION

As a result of intensive studies to solve the above problems, we accomplished the present invention on the basis of the finding that genetic polymorphisms in plant genomes are (highly) frequently found in or near transposable elements.

Accordingly, the present invention relates to a method for producing a marker for detecting a polymorphism in a plant genome, characterized in that it comprises preparing primer for nucleic acid amplification using a base sequence of transposable element and/or adjacent region thereto in plant genome. Preferably, the method of the present invention comprises:
i) preparing a pair of primers based on a base sequence of transposable element and/or adjacent region thereto present in genome of a target plant species to amplify said transposable element, or both the transposable element and the adjacent region;
ii) performing nucleic acid amplification reaction using genome DNA of the target plant species as a template; and
iii) producing a marker for detecting polymorphism in a plant genome based on polymorphism found in said nucleic acid amplification product.

Target plant species of the present invention are not specifically limited, but preferably monocotyledons such as rice, maize and barley. Rice is especially preferred.

### Transposable elements

In the method for producing a marker for detecting a DNA polymorphism in a plant genome according to the present invention, the nucleotide sequence of a transposable element present in a genome of a target plant species and adjacent regions thereto is first determined on the basis of the sequence information of a known transposable element by means of screening via Southern hybridization of a genome library and sequencing or searching through databases.

Transposable elements increase in copy number by repeating transposition in the method of biological evolution, and variations in nucleotide sequence are accumulated in the internal sequence of each copy. Therefore, transposable elements having a high homology in nucleotide sequence may be considered to be homologous, but even those having a low homology may be considered to be homologous if they have characteristic features of a class of transposable elements. For example, MITEs are characterized by their terminal inversed sequences, target sequence, presumed secondary structure and full length, etc. (T.E. Bureau et al., The Plant Cell, Vol. 6, 907-916 1994, T.E. Bureau et al., Proc. Natl. Acad, Sci. USA Vol. 91, pp. 1411-1415, 1994).

Genome libraries of plant species can be produced by known means such as the method described in Maniatis, Molecular Cloning: A Laboratory Manual, Cold Spring Harbor Laboratory, 2nd Edition, 1989, for example. Alternatively, plant genome libraries can be commercially available (e.g. rice genome libraries available from CLONTECH, maize genome libraries available from STRATAGENE, etc.).

Many sequences of plant genomes and transposable elements have been deposited in databases and those skilled in the art can search/select transposable elements that can be used for detecting polymorphisms by searching through these databases. Databases that can be used include, but not limited to, GenBank, EMBL, etc., for example.

As used herein, the term "transposable element" means a DNA unit having a defined structure that can transpose from one site to another site on DNA, as commonly understood by those skilled in the art. Therefore, "transposable elements" also include retroposons transposing via an RNA intermediate such as retrotransposons, LINES, SINEs; transposons transposing as DNA without forming any intermediate; and novel transposons recently known in plants called MITEs (Miniature Inverted Repeat Transposable Elements).

The present invention employs transposable elements present in plant genomes to detect polymorphisms in the plant genomes. Among transposable elements in plant genomes, those belonging to, but not limited to, MITEs (Miniature Inverted Repeat Transposable Elements) or SINEs (Short Interspersed Nuclear Elements) are readily employed because of their relatively short structures.

Known transposable elements falling under MITEs include, for example, transposable elements in rice such as Gaijin, Castaway, Ditto, Wanderer, Explorer, Stowaway, Tourist and Tnr2, etc. In the examples described below, primers were designed for adjacent regions to Gaijin (Gaijin-Os1, 2, 3), Castaway (Castaway-Os1, 2), Ditto (Ditto-1, 2) and Wanderer (Wanderer-Os1, 2, 3, 4) to perform PCR amplification using total DNA of rice varieties Asominori, IR24 and Kasalath as templates. A intervarietal comparison of the nucleotide sequences of the amplification products showed that polymorphisms occur with very high frequency in each transposable element sequence and adjacent regions thereto. MITE elements seem to occur in great numbers throughout rice genomes so that they can be used to produce many markers throughout rice genomes.

Of these transposable elements, Castaway was also found in maize (T. Bureau et al., Proc. Natl. Acad. Sci. USA, Vol. 93, pp. 8524-8529, August 1996), Tourist was also found in maize, sorghum and barley (T.E. Bureau et al., Proc. Natl. Acad. Sci. USA (1994) Vol. 91, pp. 1411-1415), and Stowaway was found in not only gramineous plants but also dicotyledons (T.E. Bureau et al., The Plant Cell (1994) Vol. 6, pp. 907-916). Thus, MITEs are readily presumed to show polymorphisms with high frequency in not only rice but also other plants.

Known transposable elements falling under SINEs include, for example, p-SINE1 in rice (K. Mochizuki et al., supra.). In the examples below, similar experiments to those described above for MITEs were performed on rice transposable elements p-SINE1 (p-SINE1-r1, r2, r3, r4, r5, r6 and r7) with primers used by Mochizuki et al. (1992) to amplify p-SINE1 sequences and their surrounding regions. The results showed that the frequency of intervarietal polymorphisms varies between p-SINE1 elements, some of which did not show intervarietal polymorphisms, but generally suggested that these regions are more polymorphic than genomic regions corresponding to RFLP marker probes.

The presence of as many as about 4500 copies of p-SINE1 throughout rice genomes (Ohtsubo: Cell Technology, Supplementary Volume, Plant Cell Technology Series 5, Plant Genome Science, pp. 102-113, published October 1, 1996 by Shujunsha) suggests that these elements can be used to produce many markers throughout rice genomes.

According to GenBank databases, SINEs are also found in tobacco and crucifers. Thus, SINEs are presumed to be polymorphic in not only rice but also other plants.

Transposons falling under class II other than MITEs include Ac/Ds, En/Spm and Mu and are found in many plants. In rice, for example, Tnr3 belonging to En/Spm is known, and novel elements such as Krispie, Snap, Crackle, Pop and Truncator were also recently found (W.-Y. Song et al., Mol Gen Genet (1998) Vol. 258: 449-456). Retroposons falling under class I other than SINEs are also found in many plants. In rice, for example, RIRE1 and RERE3 are known. They sometimes have an internal gene-encoding sequence, but may be rich in DNA polymorphisms in non-coding regions similarly to MITEs and SINEs. They are not as easy as MITES to use to produce markers because of their relatively long structure, but may be possibly converted into markers as described for Wanderer-Os3 in Example 2 below.

As found in the present invention, the frequency of intervarietal polymorphisms depends on the type of transposable element. Thus, PCR markers can be more efficiently produced by appropriately selecting the transposable elements used.

As used herein, the term "adjacent regions" to a transposable element are not specifically limited, but may vary with the length of the transposable element region, the type of the nucleic acid amplification reaction used, etc. In the present invention based on the finding that DNA polymorphisms frequently occur near transposable elements in plant genomes, markers for detecting polymorphisms are provided by producing a nucleic acid amplification primer pair that must be able to amplify a nucleic acid containing a transposable element or both of a transposable element and adjacent regions thereto. Thus, the distance between the primer pair is preferably within a length suitable for amplifying the nucleic acid depending on the type of the nucleic acid amplification reaction used.

As used herein, the term "adjacent regions" to a transposable element mean that an area containing a transposable element or both of a transposable element and adjacent regions thereto is within a distance suitable for nucleic acid amplification. The area containing both a transposable element and adjacent regions thereto is preferably within the range of, but not limited to, about 50 bases to about 3000 bases, more preferably about 50 bases to about 2000 bases, still more preferably about 100 bases to about 1000 bases. The adjacent regions are preferably within the range of, but not limited to, about 0 to about 3000 bases, more preferably about 0 to about 2000 bases, still more preferably about 0 to about 1000 bases on the 5' or 3' side of a transposable element.

### Nucleic acid amplification

In the present invention, a primer pair is preferably produced for amplifying a transposable element present in a genome of a target plant species or both of the transposable element and adjacent regions thereto on the basis of the determined nucleotide sequence of the transposable element and adjacent regions thereto. Then, the primer pair is used to perform a nucleic acid amplification reaction with the genomic DNA of the target plant species as a template.

The nucleic acid amplification reaction is polymerase chain reaction (PCR) (Saiki et al., 1985, Science 230, pp. 1350-1354).

The primer pair for nucleic acid amplification can be prepared by any of known methods on the basis of the nucleotide sequence of a transposable element and adjacent regions thereto. Specifically, a primer pair can be prepared, but not limited thereto, based on a base sequence of a transposable element and/or adjacent regions thereto by a method comprising generating a single-stranded DNA having the same nucleotide sequence as the nucleotide sequence of the transposable element or adjacent regions thereto or a complementary nucleotide sequence to said region or, if necessary, generating the single-stranded DNA containing a modification without affecting the binding specificity to the nucleotide sequence of the transposable element or adjacent regions thereto provided that the following conditions are satisfied:
1) length of each primer is 15-30 bases;
2) proportion of G+C content in a base sequence of each primer is 30-70%;
3) distribution of A, T, G and C is not excessively uneven in a base sequence of each primer;
4) length of nucleic acid product amplified by the pair of primers is 50-3000 bases, preferably 100-2000 bases; and
5) there is no part having complementary sequences within a base sequence of each primer itself or between the base sequences of the primers.

The number of copies in the genome of a transposable element varies from element to element, but a number of copies often occur in a genome. Primer pairs designed within a transposable element seem to be more likely to generate undesired amplification products unrelated to polymorphisms. Thus, primer pairs are preferably designed in adjacent regions to a transposable element containing a polymorphism.

However, primers may be designed in the region of a transposable element in some cases, e.g. where the region of the transposable element is too long to design primers only outside the transposable element or where the nucleic acid cannot be efficiently amplified or where a polymorphism occurs only within the transposable element and the polymorphism is used to design mismatch primers for detecting the polymorphism. Even in these cases, polymorphisms can be detected by a nucleic acid amplification reaction using primers designed in the region of the transposable element, as appropriate. In the case of Wanderer-Os3 described in Example 2 below, for example, the target site of one of the primers (SEQ ID NO: 42) wholly consists of a transposable sequence (Fig. 9), but could be used as a marker in a nucleic acid amplification reaction without any problem.

Procedures and conditions for the nucleic acid amplification reaction are not specifically limited and are well known to those skilled in the art. Appropriate conditions can be applied by those skilled in the art depending on various factors such as the nucleotide sequence of the transposable element or adjacent regions, the nucleotide sequence and length of the primer pair, etc. Generally, the nucleic acid amplification reaction can be performed under more stringent conditions (annealing reaction and nucleic acid elongation reaction at higher temperatures and less cycles) as the primer pair is longer or the proportion of G+C is higher or the distribution of A, T, G and C is evener or the number of copies of the transposable element in a genome is smaller. The use of more stringent conditions allows an amplification reaction with higher specificity.

Amplification reaction preferably involves, but not limited to, denaturation reaction at 90-95°C for 30 seconds to 2 minutes, annealing reaction at 55-65°C for 30 seconds to 2 minutes and elongation reaction at 70-75°C for 30 seconds to 2 minutes over 25-40 cycles, more preferably denaturation reaction at 94°C for 1 minute, annealing reaction at 58°C for 1 minute and elongation reaction at 72°C for 2 minutes over 30 cycles.

### Production of markers for detecting polymorphisms

After testing whether or not a polymorphism is detected in the amplification product by the nucleic acid amplification reaction with a primer pair, a marker for detecting the polymorphism is produced on the basis of the polymorphism found. Non-limiting examples of polymorphisms that can be detected in the amplification product are as follows.

### a) The polymorphism in the nucleic acid amplification product contains a type having a deleted region.

In this case, a nucleic acid amplification primer pair is prepared such that said primers are positioned on opposite sides of said deleted region to produce a marker for detecting polymorphism. Preparation of the primer pair can be performed in the same manner as described above for preliminary step i) for detecting polymorphism. The primer pair used in step i) can also be directly used as a marker for detecting the polymorphism.

If the deleted region has a sufficient size, the polymorphism can be detected from the difference in mobility by electrophoresing the amplification product on agarose gel or acrylamide gel, for example. The polymorphism can be detected when the difference in base pair numbers is about 5% or more in the case of agarose gel electrophoresis or when the difference in length is about 1 base or more in the case of sequencing acrylamide gel electrophoresis, for example. Alternatively, the polymorphism can be detected by hybridizing the nucleic acid amplification product using an oligonucleotide or a DNA fragment having a complementary sequence to the nucleotide sequence excluding the deleted region as an analytical probe. Alternatively, the polymorphism can be confirmed by determining the nucleotide sequence of the amplification product, if desired. Known techniques for electrophoresis of nucleic acids, hybridization, sequencing and the like can be used as appropriate by those skilled in the art.

In this case, the difference in the length of the amplification product directly reflects the polymorphism and markers for detecting polymorphisms on this basis are called ALP (amplicon length polymorphism) markers. This is exemplified by Gaijin-Os1 region in Example 1, Wanderer-Os2 region in Example 2 and Ditto-Os2 region in Example 4 described below. In these cases, the amplification primer pair used in step i) was directly used as a marker for detecting the polymorphism.

### b) The polymorphism in the nucleic acid amplification product contains base substitution which generates difference of recognition by restriction enzyme

In this case, a nucleic acid amplification primer pair is prepared such that said primers are positioned on opposite sides of said base substitution position to produce a marker for detecting polymorphism. Preparation of the primer pair can be performed in the same manner as described above for preliminary step i) for detecting polymorphism. The primer pair used in step i) can also be directly used as a marker for detecting the polymorphism.

In this case, a base substitution which generates a difference in restriction enzyme recognition occurs in the polymorphism of the nucleic acid amplification product, i.e. the nucleic acid amplification product may be cleaved or not with one or more specific restriction enzymes.
Thus, the amplification product can be treated with the restriction enzyme and electrophoresed on agarose gel, for example, to detect the polymorphism from the difference in mobility. The polymorphism can be confirmed by determining the nucleotide sequence of the amplification product, if desired.

In this case, the difference in the length of the restriction fragment of the amplification product by PCR or the like reflects the polymorphism and markers for detecting polymorphisms on this basis are called PCR-RFLP (PCR-restriction fragment length polymorphism) markers or CAPS (cleaved amplified polymorphic sequences) markers (A. Konieczny, F.M. Ausubel, A procedure for mapping Arabidopsis mutations using co-dominant ecotype-specific PCR-based markers, (1993), Plant J. 4(2) pp. 403-410).
This is exemplified by Gaijin-Os3 region in Example 1, Wanderer-Os1 region and -Os4 region in Example 2, Castaway-Os2 region in Example 3, Ditto-Os1 region in Example 4, and p-SINE1-r5 region and -r7 region in Example 5 described below. In Ditto-Os1 region in Example 4 among these cases, the amplification primer pair used in step i) was directly used as a marker for detecting the polymorphism.

Even if a polymorphism can be detected by the length of the nucleic acid amplification product as described in a) above, the polymorphism may be more easily detected by combination with restriction enzyme treatment in some cases, as exemplified by Wanderer-Os2 region in Example 2.

### c) The polymorphism in the nucleic acid amplification product contains base substitution which does not generate difference of recognition by restriction enzyme.

In this case, a mismatch-introducing primer pair is prepared that contains the base substitution position and modifies a region comprising the base substitution position into a base sequence which generates a difference in restriction enzyme recognition in the nucleic acid amplification product as a marker for detecting the polymorphism.

Specifically, preparation of the primer pair can be performed in the same manner as described above for preliminary step i) for detecting polymorphism. However, the primer pair used in step i) causes a polymorphism in the nucleic acid amplification product but not a difference in restriction enzyme recognition, and therefore, a mismatch is introduced into one or both of the primers to modify a region containing the base substitution position (polymorphism) into a base sequence which generates a difference in restriction enzyme recognition in the nucleic acid amplification product. As a standard technique for substituting, deleting or adding a specific nucleotide by introducing site-specific mutation via PCR, the method described in Mikaelian et al., Nucl. Acids. Res. 20:376.1992 can be used, for example. The amplification product using the mismatch-introducing primers as a marker for detecting the polymorphism may be cleaved or not with one or more specific restriction enzymes because it has a difference in restriction enzyme recognition at the mismatch-introducing site. Thus, the amplification product can be treated with the restriction enzyme and electrophoresed on agarose gel, for example, to detect the polymorphism from the difference in mobility, as described in b) above.

The introduction of a mismatch must not affect not only the binding of the primers to a target plant genome but also the polymorphic base substitution. The polymorphic base substitution is used to introduce a mismatch near it so that a difference in restriction enzyme recognition occurs by a combination of both base substitution and mismatch. Methods for introducing such a mismatch are known to those skilled in the art and described in detail in Michaels, S.D. and Amasino, R.M. (1998), Neff, M.M., Neff, J.D., Chory, J. and Pepper, A.E. (1998), for example.

Markers in this case are improvement of the CAPS markers described in b) above, and are called dCAPS (derived CAPS) markers. This is exemplified by Gaijin-Os2 region in Example 1, Wanderer-Os3 region in Example 2, Castaway-Os1 region in Example 3 and p-SINE1-r4 region in Example 5.

If there are many extra restriction sites unrelated to intervarietal polymorphisms in the case of b) or c) above, it may be difficult to identify any difference in restriction site recognition based on polymorphisms. In this case, a mismatch may be introduced into a primer as appropriate to abolish unnecessary restriction sites. For example, in Wanderer-Os3 region in Example 2, a mismatch was introduced into the 5'-primer to abolish the MseI site unrelated to polymorphisms.

### Markers for detecting polymorphisms

The present invention further provides markers for detecting polymorphisms in plant genomes produced by the method described above. According to the method of the present invention, markers for detecting polymorphisms in plant genomes can be extensively produced. Thus, markers provided by the method of the present invention are not specifically limited.

In the non-limiting examples below, polymorphisms were quite often found in a plurality of transposable elements belonging to MITEs or SINEs in rice. Thus, markers for detecting polymorphisms in rice using these transposable elements are included in the scope of the present invention. Primers having the sequences specifically disclosed herein as SEQ ID NOs: 1-2, 16-19, 22-23, 40-45, 48-49, 56-57, 58-61 and 95-100 in the Sequence Listing were actually confirmed to be usable as markers for detecting polymorphisms in rice in the examples below, and these are non-exclusively included in the scope of the present invention.

As described above, it was shown that the present invention generally makes it possible to efficiently detect polymorphisms and produce PCR markers by using transposable elements. PCR markers can be more efficiently produced by selecting the transposable element to be used because the frequency of intervarietal polymorphisms depends on the type of transposable element.

Methods for detecting polymorphisms using markers obtained by the present invention have remarkable advantages over conventional methods for detecting polymorphisms. Specifically, the frequency of intervarietal polymorphisms in nucleotide sequence is higher than obtained by PCR markers based on polymorphisms in genomic regions corresponding to RFLP probes. Thus, markers can be produced with higher frequency, and accordingly, markers can be efficiently produced. In contrast to PCR markers based on identification of RFLP-causing sites, complex steps such as screening of genomic libraries and analysis of isolated clones can be eliminated by using many transposable elements deposited in databases. Thus, markers can be efficiently produced because the time/labor for marker development can be greatly reduced (to about 1 week if all goes well). Further in contrast to microsatellite markers, polymorphisms can be detected with PCR markers by agarose gel electrophoresis that is easier than acrylamide gel electrophoresis, and the frequency of intervarietal polymorphisms in nucleotide sequence is high. Thus, easy-to-use PCR markers suitable for detecting polymorphisms on agarose gels can be produced, and the markers can be efficiently produced because they can be produced with higher frequency.

### EXAMPLES

The following examples further illustrate the present invention but are not intended to limit the technical scope of the invention. Those skilled in the art can readily add modifications/changes to the present invention on the basis of the description of the specification, and those modifications/changes are included in the technical scope of the present invention.

### Example 1: Marker development using Gaijin

### Materials and methods

Gaijin-Os1, 2 and 3 are Gaijin-Os transposable elements present in the genes OS4CL (X52623). RICCBP3 (D10985) and RICAP (D32165) submitted in databases (GenBank) (Bureau et al. 1996).

Primer pairs for amplifying the Gaijin-Os sequences and adjacent regions thereto were designed as shown in Fig. 1 (SEQ ID NOs: 1-6). These primer pairs were used to perform PCR (94°C for 1 minute, 58°C for 1 minute and 72°C for 2 minutes over 30 cycles) with genomic DNA of rice varieties of Asominori, Kasalath and IR24 as templates. The PCR products were electrophoresed and desired DNA fragments were recovered with QIAEXII (QIAGEN). Thus recovered DNA was used as template to perform sequencing reaction on a dRhodamine Terminator Cycle Sequencing FS Ready Reaction Kit (ABI) so that the nucleotide sequences were determined on ABI Model 310.

### Results and discussion

The results of a comparison of the nucleotide sequences of the PCR products of the varieties are shown in Fig. 2 - Fig. 4 and SEQ ID NOs: 7-15. Fig. 2 (SEQ ID NOs: 7-9) shows Gaijin-Os1 region, Fig. 3 (SEQ ID NOs: 10-12) shows Gaijin-Os2 region, and Fig. 4 (SEQ ID NOs: 13-15) shows Gaijin-Os3 region. In each of Fig. 2 - Fig. 4, the sequences of the 5' primer and 3' primer are placed on the 5' side and 3' side of the sequences shown in Fig. 2 - Fig. 4 in the same manner as for various amplification primer pairs in the other examples below.

### 1) Gaijin-Os1 region

When primers for Gaijin-Os1 (SEQ ID NOs: 1 and 2) were used, a polymorphism was observed in PCR product length between Asominori and Kasalath or IR24. Specifically, the PCR product length of Asominori was 434 base pairs (excluding primer regions) as compared with 278 base pairs and 277 base pairs in Kasalath and IR24, respectively, as shown in Fig. 2. This showed that the polymorphism results from the absence of Gaijin-Os1 in Kasalath and IR24 in contrast to Asominori. This polymorphism based on the difference in PCR product length was used as an ALP (Amplicon Length Polymorphism) marker. Mapping of this marker with 71 individuals of RIL (Recombinant Inbred Line) of Asominori-IR24 revealed that the locus OS4CL (Gaijin-Os1) is located on chromosome 8.

When primers for Gaijin-Os2 and Gaijin-Os3 (SEQ ID NOs: 3-4 and 5-6, respectively) were used, no polymorphism in PCR product length was observed, but polymorphisms were shown on nucleotide sequence level. The frequency of polymorphisms was compared between in the Gaijin-Os sequences and in adjacent regions to show a higher tendency in the former. However, the frequency of polymorphisms in adjacent regions to Gaijin-Os tended to be higher than in genomic sequences corresponding to conventional RFLP markers, suggesting that adjacent regions to transposable elements may be rich in intervarietal differences.

### 2) Gaijin-Os2 region

As for Gaijin-Os2 region, a marker for detecting polymorphisms was produced on the basis of polymorphisms between Asominori and Kasalath or IR24 using the dCAPS (derived cleaved amplified polymorphic sequence) method (Michaels and Amasino 1998; Neff et al. 1998).

Specifically, the nucleotide at nucleotide number 136 of the Gaijin-Os2 region of Asominori is "C" while the nucleotide at the relevant sites of Kasalath and IR24 is "T", as shown in Fig. 3 (the nucleotide numbers hereinafter used in this example refer to those of Asominori unless otherwise specified). Thus, primers shown in Fig. 5A (SEQ ID NOs: 16 and 17) were prepared as a marker for detecting polymorphisms to perform PCR (under PCR conditions of 94°C for 30 seconds, 58°C for 30 seconds and 72°C for 30 seconds over 35 cycles). The sequences of SEQ ID NOs: 16 and 17 correspond to nucleotide numbers minus 10-16 and 138-161, respectively, of the sequence of Asominori shown in Fig. 3 (SEQ ID NO: 10), but the 3' primer having the nucleotide sequence of SEQ ID NO: 17 is designed to substitute "gt" for the nucleotides "ta" at nucleotide numbers 143-144. When this marker for detecting polymorphisms is used as primers, minus 10-161 of the sequences shown in Fig. 3 (SEQ ID NOs: 10-12) is amplified, but the substitution with the 3'-primer results in the sequence "CACNNNNGTG" only in Asominori as compared with the sequence "TACNNNNGTG" in Kasalath and IR24. This nucleotide sequence "CACNNNNGTG" is recognized by restriction enzyme MslI. Thus, treatment of the amplification products with this restriction enzyme cleaved only the amplification product of Gaijin-Os2 region derived from Asominori, but not the amplification products of Kasalath and IR24. This can be used to detect polymorphisms.

### 3) Gaijin-Os3 region

As for Gaijin-Os3 region, a marker for detecting polymorphisms was produced using the normal CAPS method. Specifically, the nucleotide sequence at nucleotide numbers 355-358 of Gaijin-Os3 region of Asominori is "TTAA" while the nucleotide sequence at the relevant sites of Kasalath and IR24 is "TCAA", as shown in Fig. 4. Thus, primers shown in Fig. 5B (SEQ ID NOs: 18 and 19) were prepared as a marker for detecting polymorphisms to perform PCR (under PCR conditions of 94°C for 30 seconds, 58°C for 30 seconds and 72°C for 30 seconds over 35 cycles). The sequences of SEQ ID NOs: 18 and 19 correspond to nucleotide numbers 212-235 and 416-439, respectively, of the sequence of Asominori shown in Fig. 4 (SEQ ID NO: 13), and this marker for detecting polymorphisms is used as primers to amplify 212-439 of the sequences shown in Fig. 4 (SEQ ID NOs: 13-15). The sequence "TTAA" at nucleotide numbers 355-358 of Asominori is recognized by restriction enzyme MseI. The sequence corresponding to nucleotide numbers 401-404 of Asominori is "TTAA" in all of Asominori, Kasalath and IR24. Thus, treatment of the amplification products with this restriction enzyme cleaved the amplification product of Gaijin-Os3 region derived from Asominori at 2 sites (both 355-358 and 401-404) and the amplification products of Kasalath and IR24 at only one site (only 401-404).

Mapping with 71 individuals of RIL of Asominori-IR24 revealed that the loci RICCBP3 (Gaijin-Os2) and RICAP (Gaijin-Os3) are located on chromosome 2 and chromosome 5, respectively.

### Example 2: Marker development using Wanderer

### Materials and methods

Wanderer-Os1, 2, 3 and 4 are Wanderer-Os transposable elements present in the genes RIC3H3M (L28995), RIC3H3M (L28995), RICGLUTE (D00584) and OSALAM (X16509, S50948) submitted in databases (GenBank) (Bureau et al. 1996).

Primer pairs for amplifying the Wanderer-Os sequences and adjacent regions thereto were designed as shown in Fig. 6 (SEQ ID NOs: 20-27). These primer pairs were used to perform PCR with genomic DNA of Asominori, Kasalath and IR24 as templates, and the nucleotide sequences were determined in the same manner as described for Gaijin region in Example 1.

### Results and discussion

The results of a comparison of the nucleotide sequences of the PCR products of the varieties are shown in Fig. 7 - Fig. 10 and SEQ ID NOs: 28-39. Fig. 7 (SEQ ID NOs: 28-30) shows Wanderer-Os1 region, Fig. 8 (SEQ ID NOs: 31-33) shows Wanderer-Os2 region, Fig. 9 (SEQ ID NOs: 34-36) shows Wanderer-Os3 region, and Fig. 10 (SEQ ID NOs: 37-39) shows Wanderer-Os4 region.

### 1) Wanderer-Os2 region

When primers for Wanderer-Os2 were used, a polymorphism was observed in PCR product length between Asominori and Kasalath or IR24. Specifically, the PCR product length of Asominori was 320 base pairs (excluding primer regions) as compared with 298 base pairs and 297 base pairs in Kasalath and IR24, respectively, as shown in Fig. 8. This showed that the polymorphism results from the deletion of a part of Wanderer-Os2 in Kasalath and IR24. In the case of Wanderer-Os2, polymorphism can be detected only by the difference in PCR product length, but polymorphism can be more easily detected by combination with restriction enzyme treatment (CAPS). Specifically, the sequence at nucleotide numbers 114-119 of Kasalath and the relevant nucleotide numbers 113-118 of IR24 is "TTTAAA", which can be cleaved with restriction enzyme DraI. However, the relevant sequence of Asominori (nucleotide numbers 136-141) is "TGAAAA", which can not be cleaved with DraI. Thus, treatment of the amplification products of Wanderer-Os2 region with DraI cleaved only the amplification products of Kasalath and IR24, but not Asominori.

This polymorphism based on the difference in PCR product length of Wanderer-Os2 was used as an ALP marker. Mapping of this marker with 71 individuals of RIL of Asominori-IR24 revealed that the locus RIC3H3M (Wanderer-Os2) is located on chromosome 2.

When primers for Wanderer-Os1, Wanderer-Os3 and Wanderer-Os4 were used, no polymorphism in PCR product length was observed, but polymorphisms were shown on nucleotide sequence level. The frequency of polymorphisms was compared between in the Wanderer-Os sequences and in their adjacent regions to show a higher tendency in the former. However, the frequency of polymorphisms even in the adjacent regions to Wanderer-Os tended to be higher than in genomic sequences corresponding to conventional RFLP markers, suggesting that adjacent regions of transposable elements may be rich in intervarietal differences.

### 2) Wanderer-Os1 region

As for Wanderer-Os1 region, a marker for detecting polymorphisms was produced using the normal CAPS method. Specifically, the nucleotide sequence at nucleotide numbers 226-231 of Wanderer-Os1 region of Asominori is "TTTAAA" while the nucleotide sequence at the relevant sites of Kasalath and IR24 is "TTCAAA", as shown in Fig. 7. Thus, primers shown in Fig. 11A (SEQ ID NOs: 40 and 41) were prepared as a marker for detecting polymorphisms to perform PCR in the same manner as described in Example 1. The sequences of SEQ ID NOs: 40 and 41 correspond to nucleotide numbers 42-65 and 300-323, respectively, of the sequence of Asominori shown in Fig. 7 (SEQ ID NO: 28), and this marker for detecting polymorphisms is used as primers to amplify 42-323 of the sequences shown in Fig. 7 (SEQ ID NOs: 28-30). The sequence "TTTAAA" at nucleotide numbers 226-231 of Asominori is recognized by restriction enzyme DraI. The sequence corresponding to nucleotide numbers 197-202 of Asominori is "TTTAAA" in all of Asominori, Kasalath and IR24. Thus, treatment of the amplification products with this restriction enzyme cleaved the amplification product of Wanderer-1 region derived from Asominori at 2 sites (both 226-231 and 197-202) and the amplification products of Kasalath and IR24 at only one site (only 197-202).

### 3) Wanderer-Os3 region

As for Wanderer-Os3 region, a marker for detecting polymorphisms was produced on the basis of polymorphisms between Asominori and Kasalath or IR24 using the dCAPS method in the same manner as described for Gaijin-Os2 region.

Specifically, the nucleotide at nucleotide number 206 of Wanderer-Os3 region of Asominori is "A" while the nucleotide at the relevant sites of Kasalath and IR24 is "T", as shown in Fig. 9. Thus, primers shown in Fig. 11B (SEQ ID NOs: 42 and 43) were prepared as a marker for detecting polymorphisms to perform PCR in the same manner as described above. The sequences of SEQ ID NOs: 42 and 43 correspond to nucleotide numbers 177-205 and 269-292, respectively, of the sequence of Asominori shown in Fig. 9 (SEQ ID NO: 34), but the 5'-primer having the nucleotide sequence of SEQ ID NO: 42 is designed to substitute "T" for "A" at nucleotide number 203. When this marker for detecting polymorphisms is used as primers, 177-292 of the sequences shown in Fig. 9 (SEQ ID NOs: 34-36) are amplified, but the substitution with the 5'-primer results in "TTAA" at nucleotide numbers 203-206 of the sequence of only Asominori as compared with "TTAT" in the sequences of both Kasalath and IR24. This nucleotide sequence "TTAA" is recognized by restriction enzyme MseI. The 5'-primer (SEQ ID NO: 42) also substituted "A" for "T" at nucleotide number 185 and "T" for "A" at nucleotide number 192. These are mismatches introduced to abolish extra "TTAA" sites other than 203-206 in the amplified region. Thus, treatment of the amplification products with MseI cleaved only the amplification product of Wanderer-Os3 region derived from Asominori, but not the amplification products of Kasalath and IR24. This can be used to detect polymorphisms.

### 4) Wanderer-Os4 region

As for Wanderer-Os4 region, a marker for detecting polymorphisms was produced using the normal CAPS method. Specifically, the nucleotide sequence at nucleotide numbers 284-294 of Wanderer-Os4 region of Asominori is "Gctctgtgtgc" while the nucleotide sequence at the relevant sites of Kasalath and IR24 is "Actctgtgtgc", as shown in Fig. 10. Thus, primers shown in Fig. 11C (SEQ ID NOs: 44 and 45) were prepared as a marker for detecting polymorphisms to perform PCR in the same manner as described in Example 1. The sequences of SEQ ID NOs: 44 and 45 correspond to nucleotide numbers 52-75 and 348-371, respectively, of the sequence of Asominori shown in Fig. 10 (SEQ ID NO: 37), and this marker for detecting polymorphisms is used as primers to amplify 52-371 of the sequences shown in Fig. 10 (SEQ ID NOs: 37-39). The sequence "Gctctgtgtgc" at nucleotide numbers 284-294 of Asominori is recognized by restriction enzyme MwoI (recognizing "GCNNNNNNNGC"). The sequence corresponding to nucleotide numbers 235-245 of Asominori is "GCTCGTTTTGC" in all of Asominori, Kasalath and IR24. Thus, treatment of the amplification products with this restriction enzyme cleaved the amplification product of Wanderer-Os1 region derived from Asominori at 2 sites (both 284-294 and 235-245) and the amplification products of Kasalath and IR24 at only one site (only 235-245).

Mapping with 71 individuals of RIL of Asominori-IR24 revealed that the loci RIC3H3M (Wanderer-Os1), RICGLUTE (Wanderer-Os3) and OSALAM (Wanderer-Os4) are located on chromosome 2, chromosome 10 and chromosome 2, respectively.

### Example 3: Marker development using Castaway

### Materials and methods

Castaway-Os1 and 2 are Castaway-Os transposable elements present in the genes OSSALT (Z25811) and RICRTH (D26547) submitted in databases (GenBank) (Bureau et al. 1996).

Primer pairs for amplifying the Castaway-Os sequences and adjacent regions thereto were designed as shown in Fig. 12 (SEQ ID NOs: 46-49). These primer pairs were used to perform PCR with genomic DNA of Asominori, Kasalath and IR24 as templates and the nucleotide sequences were determined in the same manner as described in Example 1.

### Results and discussion

The results of a comparison of the nucleotide sequences of the PCR products of the varieties are shown in Fig. 13 - Fig. 14 and SEQ ID NOs: 50-55. Fig. 13 (SEQ ID NOs: 50-52) shows Castaway-Os1 region and Fig. 14 (SEQ ID NOs: 53-55) shows Castaway-Os2 region.

Castaway-Os1 was shown to be present in only Kasalath, but the presence of polymorphisms between Asominori and IR24 was also shown in its peripheral regions. Also when primers for Castaway-Os2 were used, no polymorphism in PCR product length was observed, but polymorphisms were shown on nucleotide sequence level. The frequency of polymorphisms tend to be higher than in genomic sequences corresponding to conventional RFLP markers, suggesting that transposable elements and adjacent regions thereto may be rich in intervarietal differences.

### 1) Castaway-Os1 region

As for Castaway-Os1 region, a marker for detecting polymorphisms was produced on the basis of polymorphisms between Asominori and IR24 using the dCAPS method.

Specifically, the nucleotide at nucleotide number 205 of Castaway-Os1 region of Asominori and Kasalath is "A" while the nucleotide at the relevant site of IR24 is "C", as shown in Fig. 13. Thus, primers shown in Fig. 15 (SEQ ID NOs: 56 and 57) were prepared as a marker for detecting polymorphisms to perform PCR in the same manner as described above. The sequences of SEQ ID NOs: 56 and 57 correspond to nucleotide numbers 102-125 and 209-234, respectively, of the sequence of Asominori shown in Fig. 13 (SEQ ID NO: 50), but the 3'-primer having the nucleotide sequence of SEQ ID NO: 42 is designed to substitute "g" for "a" at nucleotide number 214. When this marker for detecting polymorphisms is used as primers, 102-234 of the sequences shown in Fig. 13 (SEQ ID NOs: 50-52) is amplified, but the substitution with the 3'-primer results in "cccatgcatgg" at nucleotide numbers 204-214 of the sequence of only IR24 as compared with "cacatgcatgg" in the sequences of Asominori and Kasalath. The nucleotide sequence "CCNNNNNNNGG" is recognized by restriction enzyme BslI. The sequence corresponding to nucleotide numbers 121-131 of Asominori is "ccaaggcttgg" in all of Asominori, Kasalath and IR24. Thus, treatment of the amplification products with restriction enzyme BslI cleaved the amplification product of Castaway-Os1 region derived from IR24 at 2 sites (both 204-214 and 121-131) and the amplification products of Asominori and Kasalath at only one site (only 121-131).

### 2) Castaway-Os2 region

As for Castaway-Os2 region, a marker for detecting polymorphisms was produced using the normal CAPS method. Specifically, the nucleotide sequence at nucleotide numbers 355-365 of Castaway-Os2 region of Asominori is "CTTTTGCTTGG" while the nucleotide sequence at the relevant sites of Kasalath and IR24 is "CCTTTGCTTGG", as shown in Fig. 14. Thus, primers shown in Fig. 12B (SEQ ID NOs: 48 and 49) were directly used as a marker for detecting polymorphisms to perform PCR in the same manner as described in Example 1. The nucleotide sequence "CCTTTGCTTGG" in Kasalath and IR24 corresponding to nucleotide number 355-365 of Asominori is recognized by restriction enzyme BslI (recognizing "CCNNNNNNNGG"). Thus, treatment of the amplification products with this restriction enzyme cleaved the amplification products of Castaway-Os2 region of Kasalath and IR24, but not the amplification product derived from Asominori.

Mapping with 71 individuals of RIL of Asominori-IR24 revealed that the loci OSSALT (Castaway-Os1) and RICRTH (Castaway-Os2) are located on chromosome 1 and chromosome 7, respectively.

### Example 4: Marker development using Ditto

### Materials and methods

Ditto-Os1 and 2 are Ditto-Os transposable elements present in the genes RICHSEA (M80938) and RICOSH1 (D16507) submitted in databases (GenBank) (Bureau et al. 1996).

Primer pairs for amplifying the Ditto-Os sequences and adjacent regions thereto were designed as shown in Fig. 16 (SEQ ID NOs: 58-61). These primer pairs were used to perform PCR with genomic DNA of Asominori, Kasalath and IR24 as templates and the nucleotide sequences were determined in the same manner as described in Example 1.

### Results and discussion

The results of a comparison of the nucleotide sequences of the PCR products of the varieties are shown in Fig. 17 - Fig. 18 and SEQ ID NOs: 62-67. Fig. 17 (SEQ ID NOs: 62-64) shows Ditto-Os1 region and Fig. 18 (SEQ ID NOs: 65-67) shows Ditto-Os2 region.

### 1) Ditto-Os2 region

When primers for Ditto-Os2 were used, a polymorphism in PCR product length was observed among the 3 varieties. Specifically, the PCR product length was 267 base pairs in Asominori, 246 base pairs in Kasalath and 312 base pairs in IR24, as shown in Fig. 18. This polymorphism was shown to be result from one deletion in Asominori and 2 deletions in Kasalath within the Ditto-Os2 sequence. This polymorphism based on the difference in PCR product length was used as an ALP marker. Mapping of this marker with 71 individuals of RIL of Asominori-IR24 revealed that the locus RICOSH1 (Ditto-Os2) is located on chromosome 3.

### 2) Ditto-Os1 region

When primers for Ditto-Os1 were used, no polymorphism in PCR product length was observed, but polymorphisms were shown on nucleotide sequence level. The frequency of polymorphisms in the Ditto-Os sequence and its surrounding regions tend to be higher than in genomic sequences corresponding to conventional RFLP markers, suggesting that adjacent regions to transposable elements may be rich in intervarietal differences.

As for Ditto-Os1 region, a marker for detecting polymorphisms was produced using the normal CAPS method. Specifically, the nucleotide sequence at nucleotide numbers 206-209 of Ditto-Os1 region of Asominori is "GGCT" while the nucleotide sequence at the relevant sites of Kasalath and IR24 is "AGCT", as shown in Fig. 17. Moreover, the nucleotide sequence at nucleotide numbers 273-276 of Asominori is "agcc" while the nucleotide sequence at the relevant sites of Kasalath and IR24 is "agct". Thus, primers shown in Fig. 16A (SEQ ID NOs: 58 and 59) were directly used as a marker for detecting polymorphisms to perform PCR in the same manner as described in Example 1. The nucleotide sequence "AGCT" of Kasalath and IR24 corresponding to nucleotide numbers 206-209 and 273-276 of Asominori is recognized by restriction enzyme AluI. The sequence corresponding to nucleotide numbers 29-32 and 39-42 of Asominori is "AGCT" in all of Asominori, Kasalath and IR24. Thus, treatment of the amplification products with restriction enzyme AluI cleaved the amplification product of Ditto-Os1 region derived from Asominori at 2 sites (29-32 and 39-42) and the amplification products of Kasalath and IR24 at 4 sites (206-209 and 273-276 as well as 29-32 and 39-42).

Mapping with 71 individuals of RIL of Asominori-IR24 revealed that the locus RICHSEA (Ditto-Os1) is located on chromosome 1.

### Example 5: Marker development using p-SINE1

### Materials and methods

p-SINE1-r1 to r7 are transposable elements described by Mochizuki et al. (1992, supra.). Primer pairs for amplifying the p-SINE1 sequences (p-SINE1-r1 to -r7) and their surrounding regions were synthesized on the basis of the document of Mochizuki et al. (SEQ ID NOs: 68-73 for p-SINE1-r4, -r5 and r7). These primer pairs were used to perform PCR with genomic DNA of Asominori, Kasalath and IR24 as templates and the nucleotide sequences were determined in the same manner as described in Example 1.

### Results and discussion

The results of a comparison of the nucleotide sequences of the PCR products of the varieties are shown in Fig. 19 - Fig. 25 and SEQ ID NOs: 74-94. Fig. 19 (SEQ ID NOs: 74-76) shows p-SINE1-r1 region, Fig. 20 (SEQ ID NOs: 77-79) shows p-SINE1-r2 region, Fig. 21 (SEQ ID NOs: 80-82) shows p-SINE1-r3 region, Fig. 22 (SEQ ID NOs: 83-85) shows p-SINE1-r4 region, Fig. 23 (SEQ ID NOs: 86-88) shows p-SINE1-r5 region, Fig. 24 (SEQ ID NOs: 89-91) shows p-SINE1-r6 region, and Fig. 25 (SEQ ID NOs: 92-94) shows p-SINE1-r7 region.

As for p-SINE1-r3 region, there was no polymorphism among the 3 varieties. As for p-SINE1-r1, p-SINE1-r2 and p-SINE1-r6 regions, polymorphisms were interspersed between Asominori and Kasalath, but no polymorphism was found between Asominori and IR24. As for p-SINE1-r4, p-SINE1-r5 and p-SINE1-r7 regions, polymorphisms were observed between Asominori and IR24. The frequency of these polymorphisms was lower than in MITE regions already described, but much higher than in the conventional genomic sequences. This suggests that not only MITE regions but also p-SINE regions may be rich in intervarietal differences.

### 1) p-SINE1-r4 region

As for p-SINE1-r4 region, a marker for detecting polymorphisms was produced on the basis of polymorphisms between Asominori and Kasalath or IR24 using the dCAPS method.

Specifically, the nucleotide sequence at nucleotide numbers 82-85 of p-SINE1-r4 region of Asominori is "GGGT" while the nucleotide sequence at the relevant site of Kasalath is "AAT" and the nucleotide sequence at the relevant site of IR24 is "GGT", as shown in Fig. 22. Thus, primers shown in Fig. 26A (SEQ ID NOs: 95 and 96) were prepared as a marker for detecting polymorphisms to perform PCR in the same manner as described above. The sequences of SEQ ID NOs: 95 and 96 correspond to nucleotide numbers 60-86 and 223-246, respectively of the sequence of Asominori shown in Fig. 22 (SEQ ID NO: 83), but the 5'-primer having the nucleotide sequence of SEQ ID NO: 95 is designed to substitute "c" for "a" at nucleotide number 84. When this marker for detecting polymorphisms is used as primers, 60-246 of the sequences shown in Fig. 22 (SEQ ID NOs: 83-85) are amplified, but the substitution with the 3'-primer results in "CCGCTCAGGGG" at nucleotide numbers 84-94 of the sequence of only Asominori as compared with "CCGCTCAGAA" and "CCGCTCAGGG" in the sequences of Kasalath and IR24, respectively. The nucleotide sequence "CCGCTCAGGGG" at nucleotide numbers 84-94 of Asominori is recognized by restriction enzyme BslI (recognizing "CCNNNNNNNGG"). The sequence corresponding to nucleotide numbers 109-119 of Asominori is "CCNNNNNNNGG" in all of Asominori, Kasalath and IR24. Thus, treatment of the amplification products with restriction enzyme BslI cleaved the amplification product of p-SINE1-r4 region of Asominori at 2 sites (both 84-94 and 109-119) and the amplification products of Kasalath and IR24 at only one site (only 109-119) .

### 2) p-SINE1-r5 region

As for SINE1-r5 region, a marker for detecting polymorphisms was produced using the normal CAPS method. Specifically, the nucleotide sequence at nucleotide numbers 85-88 of p-SINE1-r5 region of Asominori is "TTAG" while the nucleotide sequence at the relevant sites of Kasalath and IR24 is "CTAG", as shown in Fig. 23. Thus, primers shown in Fig. 26B (SEQ ID NOs: 97 and 98) were prepared as a marker for detecting polymorphisms to perform PCR in the same manner as described in Example 1. The sequences of SEQ ID NOs: 97 and 98 correspond to nucleotide numbers 3-26 and 168-190, respectively of the sequence of Asominori shown in Fig. 23 (SEQ ID NO: 86), and this marker for detecting polymorphisms is used as primers to amplify 3-190 of the sequences shown in Fig. 23 (SEQ ID NOs: 86-88). The sequence "CATG" at nucleotide numbers 85-88 of Kalasath and IR24 is recognized by restriction enzyme BfaI. The sequence corresponding to nucleotide numbers 64-67 of Asominori is "CTAG" in all of Asominori, Kasalath and IR24. Thus, treatment of the amplification products with this restriction enzyme cleaved the amplification products of p-SINE1-r5 region derived from Kasalath and IR24 at 2 sites (both 85-88 and 64-67) and the amplification product of Asominori at only one site (only 85-88).

### 3) p-SINE1-r7 region

As for p-SINE1-r7 region, a marker for detecting polymorphisms was produced using the normal CAPS method. Specifically, the nucleotide sequence at nucleotide numbers 65-69 of p-SINE1-r7 region of IR24 is "cttag" while the nucleotide sequence at the relevant sites of Asominori and Kasalath is "cagtaggattag" including 7 additional nucleotides, as shown in Fig. 25. Thus, primers shown in Fig. 26C (SEQ ID NOs: 99 and 100) were prepared as a marker for detecting polymorphisms to perform PCR in the same manner as described in Example 1. The sequences of SEQ ID NOs: 99 and 100 correspond to nucleotide numbers minus 17-7 and 73-96, respectively of the sequence of Asominori shown in Fig. 25 (SEQ ID NO: 92), and this marker for detecting polymorphisms is used as primers to amplify minus 17-96 of the sequences shown in Fig. 25 (SEQ ID NOs: 92-94). The sequence "cttag" at nucleotide numbers 65-69 of IR24 is recognized by restriction enzyme Dde I (recognizing "CTNAG"). Thus, treatment of the amplification products with this restriction enzyme cleaved the amplification product of p-SINE1-r7 region derived from IR24, but not the amplification products of Asominori and Kasalath.

Mapping with 71 individuals of RIL (Recombinant inbred line) of Asominori-IR24 revealed that the loci p-SINE1-r4, p-SINE1-r5 and p-SINE1-r7 are all located on chromosome 2.

The following documents are cited herein by reference.

### References

1. Bureau, T.E., Ronald, P.C. and Wessler, S.R.(1996) A computer-based systematic survey reveals the predominance of small inverted-repeat elements in wild-type rice genes. Proc. Natl. Acad. Sci.USA 93 8524-8529.
2. Michaels, S.D. and Amasino, R.M.(1998) A robust method for detecting single-nucleotide changes as polymorphic markers by PCR. The plant Journal 14(3) 381-385.
3. Mochizuki, K., Umeda, M., Ohtsubo, H., and Ohtsubo, E.(1992) Characterization of a plant SINE, p-SINE1, in rice genome. Jpn. J. Gen. 57 155-166.
4. Neff, M.M., Neff, J.D., Chory, J. and Pepper, A.E. (1998) dCAPS, a simple technique for the genetic analysis of single nucleotide polymorphisms: experimental applications in Arabidopsis thaliana genetics. The plant Journal 14(3) 387-392.
5. P.Vos et al., Nucleic Acids Res. Vol. 23, pp. 4407-4414 (1995).
6. Ohtsubo: Cell Technology, Supplementary Volume, Plant Cell Technology Series 5, Plant Genome Science, pp. 102-113, published on October 1, 1996 by Shujunsha Co. Ltd.
7. S.R. Wessler et al., Current Opinion in Genetics & Development 1995, Vol. 5, pp. 814-821.
8. Hirochika et al., Proc. Natl. Acad. Sci. USA (1996) Vol. 93, pp. 7783-7788.
9. K. Noma et al., Genes Genet. Syst. (1997) Vol. 72, pp. 131-140.
10. H. Ohtsubo et al., Genes Genet. Syst. (1999) Vol. 74, pp. 83-91.
11. R. Motohashi et al., Mol Gen Genet (1996) Vol. 250, pp.148-152.
12. T.E. Bureau et al., Proc. Natl. Acad. Sci. USA (1994) Vol. 91, pp. 1411-1415.
13. T.E. Bureau et al., The Plant Cell (1994) Vol. 6, pp. 907-916.
14. N. Huang et al., Gene (1992) Vol. 111, pp. 223-228.
15. F. Vignols et al., Gene (1994) Vol. 142, pp. 265-270.
16. K. Mochizuki et al., Jpn.J.Genet. (1993) Vol. 68, pp. 25-217.
17. W.-Y. Song et al., Mol Gen Genet (1998) Vol. 258: 449-456.
18. T. Tenzen et al., Mol Gen Genet (1994) Vol. 245: 441-448.

## Claims

1. A method for producing a marker for detecting polymorphism in a plant genome, wherein the method comprises preparing primer for nucleic acid amplification using on a base sequence of transposable element and/or adjacent region thereto in plant genome.

2. The method for producing a marker for detecting polymorphism of Claim 1, comprising
i) preparing a pair of primers based on a base sequence of transposable element and/or adjacent region thereto present in genome of a target plant species to amplify said transposable element, or both the transposable element and the adjacent region;
ii) performing nucleic acid amplification reaction using genome DNA of the target plant species as a template; and
iii) producing a marker for detecting polymorphism in a plant genome based on polymorphism found in said nucleic acid amplification product.

3. The method for producing a marker for detecting polymorphism of Claim 2, wherein the step iii) for producing a marker comprises any one of the following a)-c):
a) if said polymorphism in nucleic acid amplification product contains a type having a deleted region, preparing a pair of primers for nucleic acid amplification such that said primers are positioned on opposite sides of said deleted region to produce a marker for detecting polymorphism;
b) if said polymorphism in nucleic acid amplification product contains base substitution which generates difference of recognition by restriction enzyme, preparing a pair of primers for nucleic acid amplification such that said primers are positioned on opposite sides of said base substitution position to produce a marker for detecting polymorphism; or
c) if said polymorphism in said nucleic acid amplification product contains base substitution which does not generate difference of recognition by restriction enzyme, preparing a pair of primers for introducing mismatch such that said primers comprises said base substitution position and modifies a region comprising said base substitution position to a base sequence which generates difference of recognition by restriction enzyme for the nucleic acid amplification product, to produce a marker for detecting polymorphism.

4. The method for producing a marker for detecting polymorphism of Claim 2 or 3, wherein the step i) and iii) of preparing the pair of primers meet the following conditions of:
1) length of each primer is 15-30 bases;
2) proportion of G+C content in a base sequence of each primer is 30-70%;
3) distribution of A, T, G and C is not excessively uneven in a base sequence of each primer;
4) length of nucleic acid product amplified by the pair of primers is 50-2000; and
5) there is no part having complementary sequences within a base sequence of each primer itself or between the base sequences of the primers.

5. The method for producing a marker for detecting polymorphism of any one of Claims 1-4, wherein the nucleic acid amplification of the step ii) is performed by polymerase chain reaction.

6. The method for producing a marker for detecting polymorphism of any one of Claims 1-5, wherein said target plant is monocot.

7. The method for producing a marker for detecting polymorphism of any one of Claims 1-6, wherein said target plant is rice.

8. The method for producing a marker for detecting polymorphism of any one of Claims 1-7, wherein said transposable element is MITE (Miniature Inverted Repeat transposable Element) or SINE (Short Interspersed Nuclear Element).

9. A marker for detecting polymorphism produced by the method of any one of Claims 1-8.


SEQUENCE LISTING

<110> JAPAN TOBACCO INC.
      Syngenta Limited
<120> Markers for detecting genetic polymorphism in plant based
        on transposable element and a method for producing thereof
<130> YCT 638
<160> 100
<170> PatentIn Ver. 2.0

<210> 1
<211> 24
<212> DNA
<213> Artificial Sequence
<220>
<223> Description of Artificial Sequence: primer
<400> 1
aacgtgtgaa ggactcaaga agcc                                        24

<210> 2
<211> 24
<212> DNA
<213> Artificial Sequence
<220>
<223> Description of Artificial Sequence: primer
<400> 2
cctcagatgt tgacttgagc caac                                        24

<210> 3

<211> 24
<212> DNA
<213> Artificial Sequence
<220>
<223> Description of Artificial Sequence: primer
<400> 3
ctgatgctaa tgctgcgaaa agtt 24

<210> 4
<211> 23
<212> DNA
<213> Artificial Sequence
<220>
<223> Description of Artificial Sequence: primer
<400> 4
atgtgtgttt tcggcatggt ttg 23

<210> 5
<211> 24
<212> DNA
<213> Artificial Sequence
<220>
<223> Description of Artificial Sequence: primer
<400> 5
attcgtagga aaggcttcac ttgc 24

<210> 6
<211> 24
<212> DNA

<213> Artificial Sequence
<220>
<223> Description of Artificial Sequence: primer
<400> 6
cgtgctttgc gtttagaggg tcgg 24

<210> 7
<211> 434
<212> DNA
<213> Rice
<400> 7
   cttcttttca cgtttttatt taggttttga tacaatatat acgcaatagg tgttgttttt 60
   taaaaaaaat aaacaaaatg gacattacat tattggctgt gtttagatcc aaaatttaga 120
   tccaaacttc aatccttttc catcacatca acctgtcata cacacacaac ttttcagtca 180
   catcatctcc aatttcaacc aaaatccaaa ctttggatcc aactaaacac aaccattgtt 240
   attattatta ttataaactt ataaaaagga catgaaaaca attaatgaaa ctaaggacag 300
   atttcatttc actgacatgc ttagaaagta tagttgccaa tatatgttat ctttttaatg 360
   cgtgtgtgtg tgtgtgtaaa gtggagctcc actcccacac acaaacatga tggcatgact 420
   atttaataag ctaa 434

<210> 8
<211> 278
<212> DNA
<213> Rice
<400> 8
cttcttttca cgtttttatt tagttttaga tactacaata tacacaatag gtgttgtttt 60
tttaaaaaat aaacaaaatg gacattacat tgttgttatt attattataa acttataaaa 120
aggacatgaa aacaattaag gaaactaagg acaaaattca tttcactgac atgtttagaa 180
agtatagttg ccaatatatg gaacttttta atgtgtgttt agagtggagc tccactccca 240

cacacaaaca tgatggcatg actatttaat aagctaaa 278

<210> 9
<211> 277
<212> DNA
<213> Rice
<400> 9
cttcttttca cgtttttatt tagttttaga tactacaata tacacaatag gtgttgtttt 60
tttaaaaaat aaacaaaatg gacattacat tgttgttatt attattataa acttataaaa 120
aggacatgaa aacaattaag gaaactaagg acaaaattca tttcactgac atgtttagaa 180
agtatagttg ccaatatatg gaacttttta atgtgtgttt agagtggagc tccactccca 240
cacacaaaca tgatggcatg actatttaat aagctaa 277

<210> 10
<211> 301
<212> DNA
<213> Rice
<400> 10
ttatgatact actagggttg tgtttagatc caaactttca actttttcca tcacatcaac 60
ctgtcataca cacataactt ttcagtcaca tcgtactaat ttcaacccaa atttctaact 120
ttagaaggaa ctaaacacag cctagcttct ctagcacagc agcatgttca cttcttaatt 180
agtggcatct gaaccatgtg atttttgaaa gtgaaactat tattaactga tcgagttgcc 240
gagataagga cagctccatg ttaaggtggg gcatcacatt ctccagcctt tcaagcaacc 300
c 301

<210> 11
<211> 299
<212> DNA
<213> Rice

<400> 11

ttatgatact actagggttg tgtttagatc caaactttca actttttcca tcacatcaac 60

ctgtcataca cacaactttt cagtcacatc gtaccaattt caacccaaac ttctaacttt 120

ggaaggaact aaatacagcc tagcttctct agcacagcag catgttcact tcttaattag 180

tggcatctga accatgtgat ttttgaaagt gaaactattg ttaactgatc gagttgccga 240

gataaggaca gctccatgtt aaggtggggc atcacattct ccagcctttc aagcaaccc 299

<210> 12

<211> 301

<212> DNA

<213> Rice

<400> 12

ttatgatact actagggttg tgtttagatc taaactttca actttttcca tcacatcaac 60

ctgtcataca cacacaactt ttcagtcaca tcgtaccaat ttcaacccaa atttctaact 120

ttagaaggaa ctaaatacag cctagcttct ctagcacagc agcatgttca cttcttaatt 180

agtggcatct gaaccatgtg atttttgaaa gtgaaactat tattaactga tcgagttgcc 240

gagataagga cagctccatg ttaaggtggg gcatcacatt ctccagcctt tcaagcaacc 300

c 301

<210> 13

<211> 468

<212> DNA

<213> Rice

<400> 13

aaatcattgc tactcctgct ttcaatacag tactactgct gctggtaaat gcagatttga 60

tattggaaat ggcctattgg cttgtattta aatcttaagc attttcttaa caggatgtac 120

tgacttaatc atcaatgcag catgttgttt atgaaaaaaa tattgctata tttttgtagc 180

tagttgtttc tgtgaaggtg aaaatctgag cagggaatat ctcggtgaag gaagcaaaaa 240

cctcatgctc tgaatgaccc attttctttg acagagccct tgtttagttt ctaacttttt 300

cttcaaactt tcaacttttt catcacatca aaactttcct atacacataa acttttaact 360
tttccgttgt atcgttccaa tttcaaccaa actttcaatt ttaacgtgaa ttgaacacac 420
cctctgaatg accattttaa tagttaatgt atcatgtaat gagatatt 468

<210> 14
<211> 468
<212> DNA
<213> Rice
<400> 14
aaatcattgc tactcctgct ttcaatacag tactactgct gctggtaaat gcagatttga 60
tattggaaat ggcatattgg cttgtattta aatcttaagc tttttcttaa caggatgtac 120
tgacttaatc atcaatgcag cgtgttgttt atgaaaaaaa tattgctata tttttgtagc 180
tagttgtctc tgtgaaggtg aaaatctgag cagggaatat ctcggtgaag gaagcaaaaa 240
cctcatgctc tgaatgaccc cttttctttg acagagccct tgtttagttt ctaacttttt 300
cttcaaactt tcaacttttt catcacatca aaacttttct atacacataa actttcaact 360
tttccgttat atcgtttcaa tttcaaccaa actttcaatt ttaacgtgaa ttgaacacac 420
cctctgaatg accattttaa tagttaatgt atcatgtaat gagatatt 468

<210> 15
<211> 469
<212> DNA
<213> Rice
<400> 15
aaatcattgc tactcctgct ttcaatacag tactactgct gctggtaaat gcagatttga 60
tattggaaat ggcctattgg cttgtattta aatcttaagc attttcttaa caggatgtac 120
tgacttaatc atcaatgcag catgttgttt atgaaaaaaa atattgctat atttttgtag 180
ctagttgttt ctgtgaaggt gaaaatctga gcagggaata tctcggtgaa ggaagcaaaa 240
acctcatgct ctgaatgacc ccttttcttt gacagagccc ttgtttagtt tctaaatttt 300
tcttcaaact ttcaactttt tcatcatatc aaaacttttc tatacacata aattttcaac 360

ttttccatta tatcgttcta atttcaacca aacttctaat tttaacgtga attgaacaca 420
acctctgaat gaccatttta atagttaatg tatcatgtaa tgagataat 469

<210> 16
<211> 27
<212> DNA
<213> Artificial Sequence
<220>
<223> Description of Artificial Sequence: primer
<400> 16
gcgaaaagtt ttatgatact actaggg 27

<210> 17
<211> 24
<212> DNA
<213> Artificial Sequence
<220>
<223> Description of Artificial Sequence: primer
<400> 17
tgctgtgcta gagaagcacg gctg 24

<210> 18
<211> 24
<212> DNA
<213> Artificial Sequence
<220>
<223> Description of Artificial Sequence: primer
<400> 18
agggaatatc tcggtgaagg aagc 24

<210> 19
<211> 24
<212> DNA
<213> Artificial Sequence
<220>
<223> Description of Artificial Sequence: primer
<400> 19
taaaatggtc attcagaggg tgtg                                                24

<210> 20
<211> 24
<212> DNA
<213> Artificial Sequence
<220>
<223> Description of Artificial Sequence: primer
<400> 20
tggagtgtag aggctggaat gata                                                24

<210> 21
<211> 24
<212> DNA
<213> Artificial Sequence
<220>
<223> Description of Artificial Sequence: primer
<400> 21
aaatctgctg gacctacacc gatt                                                24

<210> 22

<211> 24
<212> DNA
<213> Artificial Sequence
<220>
<223> Description of Artificial Sequence: primer
<400> 22
tatgacaaag attctgtgtt cggc                                                24

<210> 23
<211> 24
<212> DNA
<213> Artificial Sequence
<220>
<223> Description of Artificial Sequence: primer
<400> 23
ttaggcacaa tagattcgtc tcgc                                                24

<210> 24
<211> 24
<212> DNA
<213> Artificial Sequence
<220>
<223> Description of Artificial Sequence: primer
<400> 24
gacttttgg acttacactg ctac                                                24

<210> 25
<211> 24
<212> DNA

<213> Artificial Sequence
<220>
<223> Description of Artificial Sequence: primer
<400> 25
tgttttattt gggttgccta cacc 24

<210> 26
<211> 24
<212> DNA
<213> Artificial Sequence
<220>
<223> Description of Artificial Sequence: primer
<400> 26
tatgtaaatg atgggtgcgg ttgc 24

<210> 27
<211> 24
<212> DNA
<213> Artificial Sequence
<220>
<223> Description of Artificial Sequence: primer
<400> 27
actggattgg atggataaca gccg 24

<210> 28
<211> 422
<212> DNA
<213> Rice
<400> 28

tccattatct aaaaaatgta tactttattg tggaaatttt catttgagac aatcacgcag 60
cttgcttatc taaggcccca tttaatctcc acatttggag ataaattttg gttgcacgta 120
aaacgagaaa gctcattagc acataattaa ttaagtatta actattataa atttgaaaaa 180
tggatttatt ttctttttta aaacaacttc tatatagaaa ctttttttaa aaaaatgtat 240
catttaacaa tttgaaaagc gtgctaacgg aaaacaagga agttgaagaa tagaacaggg 300
cctaagtcgt tcttacatca tccttttttt ttcttttttg ctctattgtt tcgcttcctt 360
agtagaacca ataactccat atagaggtca tcttctatta gaattgacat aaaaactgtt 420
tt 422

<210> 29
<211> 424
<212> DNA
<213> Rice
<400> 29
tccattatct aaaaaatgta tactttattg tggaaatttt catttgagac aatcacgcag 60
cttgcttatc taaggcccca tttaatctcc acatttggag ataaattttg gttgcacgta 120
aaacgagaaa gctcattagc acataattaa ttaagtatta actattataa atttgaaaaa 180
tggatttatt ttctttttta aaacaacttc tatatagaaa cttttttcaa aaaaaatgta 240
tcatttaaca atttgaaaag cgtgctaacg gaaaacaagg aagttgaaga atagaacagg 300
gcctaagtcg ttcttacatc atcctttttt ttttcttttt tgctctattg tttcgcttcc 360
ttagtagaac caataactcc atatagaggt catcttctat tagaattgac ataaaaactg 420
tttt 424

<210> 30
<211> 423
<212> DNA
<213> Rice
<400> 30
tccattatct aaaaaatgta tactttattg tggaaatttt catttgagac aatcacgcag 60

cttgcttatc taaggcccca tttaatctcc acatttggag ataaattttg gttgcacgta 120
aaacgagaaa gctcattagc acataattaa ttaagtatta actattataa atttgaaaaa 180
tggatttatt ttctttttta aaacaacttc tatatagaaa cttttttcaa aaaaaatgta 240
tcatttaaca atttgaaaag cgtgctaacg gaaaacaagg aagttgaaga atagaacagg 300
gcctaagtcg ttcttacatc atcctttttt tttctttttt gctctattgt ttcgcttcct 360
tagtagaacc aataactcca tatagaggtc atcttctatt agaattgaca taaaaactgt 420
ttt 423

<210> 31
<211> 320
<212> DNA
<213> Rice
<400> 31
actcactgct gggaacatat atgttgcgca cgaaaaatag agcggtccat tagcgcgtga 60
ttagttaagt attagttttt tccttcaaaa atgaatcaat atgatttttt aagcagcttt 120
tgtatagaaa ctttttgaaa aaaaatacca tttaacagtt tgaaaagcat atgcgcggaa 180
tatgagagag aggtgttggg aaccaaatgt accgaactgg gggtttggat ggaaccatgt 240
cacatcagat gttggacact aattagatgt attaaacgta gattattgac aaaatccatt 300
ccataactct aaactaatta 320

<210> 32
<211> 298
<212> DNA
<213> Rice
<400> 32
actcactgct gggaacatat acgttgcaca cgaaaaatag agcggtccat tagcacatga 60
ttagttaagt tttagttttg attttttaag cagctttcgt atagaaactt ttttttaaaa 120
aaataccgtt taacagtttg aaaagcatat gcgcggaaca cgagagagag gtgttgtgta 180
ccaagtgtac cgaactgggg gtttggatgg aaccatgtca catcggatgt tggacactaa 240

ttagatgtat taaacgtaga ttattgacaa aatccattcc ataactctaa actaatta 298

<210> 33
<211> 297
<212> DNA
<213> Rice
<400> 33
actcactgct gggaacatat atgttgcaca cgaaaaatag agcggtccat tagcacgtga 60
ttagttaagt tttagttttg attttttaag cagctttcgt atagaaactt tttttaaaaa 120
aataccgttt aacagtttga aaagcatatg cgcggaaaac gagagagagg tgttgtgtac 180
caagtgtacc gaactggggg tttggatgga accatgtcac atcggatgtt ggacactaat 240
tagatgtatt aaacgtagat tattgacaaa atccattcca taactctaaa ctaatta 297

<210> 34
<211> 475
<212> DNA
<213> Rice
<400> 34
ctttaagtaa caatcatgag caatagtgtt gcaatgatat ttaggctgca ttcgtttact 60
ctcttgattt ccatgagcac gcttcccaaa ctgttaaact ctgtgttttt tgccaaaaaa 120
aaatgtatag gaaagttgct tttaaaaaat catatcaatc cattttttaa gttatagcta 180
atacttaatt aatcatgcgc taataagtca ctctgttttt cgtactagag agattgtttt 240
gaaccagcac tcaagaacac agccttaacc cagccaaata atgctacaac ctaccagtcc 300
acacctcttg taaagcattt gttgcatgga aaagctaaga tgacagcaac ctgttcagga 360
aaacaactga caaggtcata gggagaggga gcttttggaa aggtgccgtg cagttcaaac 420
aattagttag cagtagggtg ttggtttttg ctcacagcaa taagaagtta atcat 475

<210> 35
<211> 474

<212> DNA
<213> Rice
<400> 35
ctttaagtaa caatcatgag caatagtgtt gcaatgatat ttaggctgca ttcgtttact 60
ctcttgattt ccatgagcac gcttcccaaa ctgttaaact ctgtgttttt tgccaaaaaa 120
aatgtatagg aaagttgctt ttaaaaaatc atatcaatcc attttttaag ttatagctaa 180
tacttaatta atcatgcgct aatatgtcac tctgtttttc gtactagaga gattgttttg 240
aaccagcact caagaacaca gccttaaccc agccaaataa tgctacaacc taccagtcca 300
cacctcttgt aaagcatttg ttgcatggaa aagctaagat gacagcaacc tgttcaggaa 360
aacaactgac aaggtcatag ggagagggag cttttggaaa ggtgccgtgc agttcaaaca 420
attagttagc agtagggtgt tggtttttgc tcacagcaat aagaagttaa tcat 474

<210> 36
<211> 474
<212> DNA
<213> Rice
<400> 36
ctttaagtaa caatcatgag caatagtgtt gcaatgatat ttaggctgca ttcgtttact 60
ctcttgattt ccatgagcac gcttcccaaa ctgttaaact ctgtgttttt tgccaaaaaa 120
aatgtatagg aaagttgctt ttaaaaaatc atatcaatcc attttttaag ttatagctaa 180
tacttaatta atcatgcgct aatatgtcac tctgtttttc gtactagaga gattgttttg 240
aaccagcact caagaacaca gccttaaccc agccaaataa tgctacaacc taccagtcca 300
cacctcttgt aaagcatttg ttgcatggaa aagctaagat gacagcaacc tgttcaggaa 360
aacaactgac aaggtcatag ggagagggag cttttggaaa ggtgccgtgc agttcaaaca 420
attagttagc agtagggtgt tggtttttgc tcacagcaat aagaagttaa tcat 474

<210> 37
<211> 435
<212> DNA

<213> Rice

<400> 37

acctaggtga aaaaaaatac atattcgcca caactcgcaa catgtaccaa ttcagcagca 60

agtgtaagag agaagatttc tctcgtttta cacgcgcacc gttcaattcc tgaactacta 120

aacggtatga ttttttgcaa aaattttcta taggaaagtt acttaaaaat tatattaatc 180

tatttttaaa atttaaaata gttaatactc aattaattat acgttaatgg ctcagctcgt 240

tttgcgtaca ttctcaatcg attcttttcc tctgctctca aatgctctgt gtgcgatcag 300

gtattcatgt tcagctcgca caagcacaag caagacagat ggaattccta ctgacctgcg 360

ccttttgagt gctccaactc tcaaagtctc aaggccatta aattgcctat gggctcacca 420

gccaataaca aactc 435

<210> 38

<211> 433

<212> DNA

<213> Rice

<400> 38

acctaggtga aaaaaaatac atattcgcca caactcgcaa catgtaccaa ttcagcagca 60

agtgtaagag agaagatttc tctcgtttta cacgcgcacc gttcaattcc tgaactacta 120

aacggtatga tttttgcaa aaattttcta taggaaagtt acttaaaaat tatattaatc 180

tatttttaaa atttaaaata gttaatactc aattaatcat acgttaatgg ctcagctcgt 240

tttgcgtact ctcaatcgat tcttttcctc tgctctcaaa tactctgtgt gcgatcaggt 300

attcatgttc agctcgcaca agcacaagca agacagatgg aattcctact gacctgcgcc 360

ttttgagtgc tccaactctc aaagtctcaa ggccattaaa ttgcctatgg gctcaccagc 420

caataacaaa ctc 433

<210> 39

<211> 433

<212> DNA

<213> Rice

<400> 39

acctaggtga aaaaaaatac atattcgcca caactcgcaa catgtaccaa ttcagcagca 60

agtgtaagag agaagatttc tctcgtttta cacgcgcacc gttcaattcc tgaactacta 120

aacggtatga ttttttgcaa aaattttcta taggaaagtt acttaaaaat tatattaatc 180

tatttttaaa atttaaaata gttaatactc aattaatcat acgttaatgg ctcagctcgt 240

tttgcgtact ctcaatcgat tcttttcctc tgctctcaaa tactctgtgt gcgatcaggt 300

attcatgttc agctcgcaca agcacaagca agacagatgg aattcctact gacctgcgcc 360

ttttgagtgc tccaactctc aaagtctcaa ggccattaaa ttgcctatgg gctcaccagc 420

caataacaaa ctc 433

<210> 40

<211> 24

<212> DNA

<213> Artificial Sequence

<220>

<223> Description of Artificial Sequence: primer

<400> 40

atttgagaca atcacgcagc ttgc 24

<210> 41

<211> 24

<212> DNA

<213> Artificial Sequence

<220>

<223> Description of Artificial Sequence: primer

<400> 41

ggatgatgta agaacgactt aggc 24

<210> 42

<211> 29
<212> DNA
<213> Artificial Sequence
<220>
<223> Description of Artificial Sequence: primer
<400> 42
gctaatacat aattattcat gcgctatta                                        29

<210> 43
<211> 24
<212> DNA
<213> Artificial Sequence
<220>
<223> Description of Artificial Sequence: primer
<400> 43
aggttgtagc attatttggc tggg                                             24

<210> 44
<211> 24
<212> DNA
<213> Artificial Sequence
<220>
<223> Description of Artificial Sequence: primer
<400> 44
tcagcagcaa gtgtaagaga gaag                                             24

<210> 45
<211> 24
<212> DNA

<213> Artificial Sequence
<220>
<223> Description of Artificial Sequence: primer
<400> 45
cactcaaaag gcgcaggtca gtag 24

<210> 46
<211> 22
<212> DNA
<213> Artificial Sequence
<220>
<223> Description of Artificial Sequence: primer
<400> 46
gaggcttctt tggcagacgt gg 22

<210> 47
<211> 22
<212> DNA
<213> Artificial Sequence
<220>
<223> Description of Artificial Sequence: primer
<400> 47
gtagttgtgt agggcaatgg gg 22

<210> 48
<211> 24
<212> DNA
<213> Artificial Sequence
<220>

<223> Description of Artificial Sequence: primer

<400> 48

actccactcc aaacctaact ccta                                          24

<210> 49

<211> 24

<212> DNA

<213> Artificial Sequence

<220>

<223> Description of Artificial Sequence: primer

<400> 49

gaacagacat ggcaatttac ggct                                          24

<210> 50

<211> 287

<212> DNA

<213> Rice

<400> 50

cgctgagtga gcttggctcg cttggtcaaa ctccgtgcag ggacattcag ttagctagct 60

agcagcattg tcgacaataa gatagccttt aaatgttagc actcaccagc ttgtcaaaaa 120

ccaaggcttg gtgacggcgg cttcagaatg aaggatagat ggataaatgt ctagaatatt 180

ataaagtcca acaaaagatg gagcacatgc atgaaagatt acgtacacga atgcagttga 240

tacagtggat gttaggcata agaagcacta taaatagagg gtgcaat                 287

<210> 51

<211> 772

<212> DNA

<213> Rice

<400> 51

cgctgagtga gcttggctcg cttggtcaaa ctccgtgcag ggacattcag ttagctagca 60
gcattatcga taataaggta gtctttaagg gcccatttaa atcgtaggaa taagaaaaca 120
tagaaatagg aaaaacgcag gattttaata ggaatgtaag tgtaaaacag aggattgcaa 180
aacacaggaa aaatagagga atggtctttg gattggaccg caggaaaaac gcaggaatta 240
gaggagagat aaagactcaa aggaaagttt ccaagagatt ttacctcatg ttagaattcc 300
tccaaaactt acatggcatt agccaatcca taggaatttc ataggattcc ataagatcca 360
ttcctttgat tcaaatggct atataggaaa aaatcctata ggaatgaaat cctctaaaat 420
tcctatgaat ttcctttgaa tcaaaggggg cctaaatgtt acttactccg tttcacaata 480
taagactttc tagcattatc catatacata tagatgttaa tgaatctaaa catatgtata 540
tggataatgc tagaaagtct tacattgtga aacggatgga gtagcactca ccagcttgtc 600
aaaaaccaag gcttggtgac ggcggcttca gaacgaagga tagatggata aatgtctaga 660
atattataaa gtccaacaaa agatggagca catgcatgaa agattacgta cacgaatgca 720
gttgatacag tggatgttag gcataagaag cactataaat agagggtgca at 772

<210> 52
<211> 287
<212> DNA
<213> Rice
<400> 52
cgctgagtga gcttggctcg cttggtcaaa ctccgtgcag ggacattcag ttagctagct 60
agcagcattg tcgacaataa gatagccttt aaatgttagc actcaccagc ttgtcaaaaa 120
ccaaggcttg gtgacggcgg cttcagaatg aaggatagat ggataaatgt ctagaatatt 180
ataaagtcca acaaaagatg gagcccatgc atgaaagatt acgtacacga atgcagttga 240
tacagtggat gttaggcata agaagcacta taaatagagg gtgcaat 287

<210> 53
<211> 581
<212> DNA
<213> Rice

<400> 53

gagctaaaat taagtgtccc tttgaatcac acgatttaaa aaacaaaggt atagggaaac 60

ataggattca gacaggaatg caagtctaaa ataaaggatt gcaaaacata ggaaaaacac 120

aggaatgatc gtttgattgt tggatcacag taaaaataca tgaattagat aaagagagat 180

agtcttaaaa gaaattttct aaaaggttag agtttttgct aagtttcctc caaaatctat 240

atgcaatgag ccattctata ggaatttcat atgatttaaa aagtttcaat cctttaaatc 300

aaataaccac gtagaaaaaa agttatatat gagtatgaaa ttacaacgta gaatcttttg 360

cttggggccc taagagtgac actaccaaac aagtcctact tctatgctaa gcgaaacgac 420

atatttacga acacaaaata atttgtaaat aaaactttta tatacgtgta gcgatctaaa 480

taaacactga aaatttaaat ttcaataaaa aaccctaaaa tcaactttaa atttaacact 540

gaaaatttaa atttgggcta ataaacatat gcaaaagtta a 581

<210> 54

<211> 575

<212> DNA

<213> Rice

<400> 54

gagctaaaat taagtgtccc tttgaatcac acgatttaaa aaacaaaggt atagggaaac 60

ataggattca gacaggaatg caagtctaaa ataaaggatt gcaaaacata ggaaaaacac 120

aggaatgatc gtttgattgt tggatcacag taaaaataca tgaattagat aaaaagagat 180

agtcttaaaa gaaattttct aaaaggttag agtttttgct aagttttctc caaaatttat 240

atgcaatgag caaatttcat atgatttaaa aagtttcaat cctttaaatc aaataaccac 300

gtagaaaaaa agtcatatat gagtatgaaa ttacagcgta gaatcctttg cttggggccc 360

taagagtgac actctaccaa acaagtccta cttctatgct aagcgaaacg atatatttac 420

gaacacaaaa tataatttgt gaataaaact tttatatacg tgtagcgatc taaataaaca 480

ctgaaaattt aaatttcaat aaaaaaccct aaaatcaact ttaaatttaa cactgaaaat 540

ttaaatttgg gctaataaac atatgcaaaa gttaa 575

<210> 55

<211> 583
<212> DNA
<213> Rice
<400> 55
gagctaaaat taagtgtccc tttgaatcac acgatttaaa aaacaaaggt atagggaaac 60
ataggattta gacaggaatg caagtctaaa ataaaggatt acaaaacata agaaaaacaa 120
aggaatgatc gtttgattgt tggatcacag taaaaataca tgaattagat aaagagagat 180
agtcttaaaa gaaattttct aaaaggttag agtttttgct aagtttcctc caaaatctat 240
atgcaatgag ccattctata ggtatttcat atgatttaaa aagtttcaat cctttaaatc 300
aaataaccac gtagaaaaaa agtcatatat gagtatgaaa ttacaacgta gaatcctttg 360
cttggggccc taagagtgac actctaccaa acaagtccta cttctatgct aagcgaaacg 420
acatatttac gaacacaaaa taatttgtga ataaaacttt tatatacgtg tagcgatcta 480
aataaacact gaaaatttaa atttcaataa aaaaccctaa aatcaacttt aaatttaaca 540
ctgaaaattt aaatttgggc taataaacat atgcaaaagt taa 583

<210> 56
<211> 24
<212> DNA
<213> Artificial Sequence
<220>
<223> Description of Artificial Sequence: primer
<400> 56
ctcaccagct tgtcaaaaac caag 24

<210> 57
<211> 26
<212> DNA
<213> Artificial Sequence
<220>

<223> Description of Artificial Sequence: primer
<400> 57
gcattcgtgt acgtaatctt ccatgc 26

<210> 58
<211> 24
<212> DNA
<213> Artificial Sequence
<220>
<223> Description of Artificial Sequence: primer
<400> 58
cgttattatg gggtgaaaag atgg 24

<210> 59
<211> 24
<212> DNA
<213> Artificial Sequence
<220>
<223> Description of Artificial Sequence: primer
<400> 59
ccgttactac aagcctataa caca 24

<210> 60
<211> 24
<212> DNA
<213> Artificial Sequence
<220>
<223> Description of Artificial Sequence: primer
<400> 60

agcttttcct ctctacctct aatg 24

<210> 61
<211> 23
<212> DNA
<213> Artificial Sequence
<220>
<223> Description of Artificial Sequence: primer
<400> 61
ccttgttgtt taccttgagc tga 23

<210> 62
<211> 544
<212> DNA
<213> Rice
<400> 62
ttttgccctt aagagcaggt ataatagcaa gcttaagtca gctataaaca tattttaaag 60
agataaaaaa agatagagaa gagcagcggg ctacagatct gtagtcagct gcagcacgga 120
cttcaatacg taatgtatgt atgataggtg ggaccaggta ttaatagtac agtaagcaac 180
tattgtatga attgactatt acattggcta tagatgattt taagttagta gtgggctata 240
ctattaaact tgctctaatt gttttatgcc agagccataa ttttatcctt tcttttgagg 300
aataaatttt atactacgga gtaacttatt acgcgattca cggttgaata tctgtttact 360
tatcttttta cttatactta tatttatcaa tcaaaattta aaatttcaac tttaaatttg 420
aagttgattt tgggggtttt gatcaaaatt catttttcag tctttgcttt aagatcacta 480
aaaacacgta tatacaagtt ttattaataa attattcttt tttggcaaat atgctgtttg 540
caaa 544

<210> 63
<211> 561

<212> DNA
<213> Rice
<400> 63
ttttgccctt aagagcaggt ataatagcaa gctataagcc agctataaac atattttaaa 60
aagataaaag aagatagaga agagcagcgg gctacagatc tgtagtcagc tgcagcacgg 120
actccaatac gtaatgtatg tatgataggt ggaaccaggt attaatagta cagtaagcaa 180
ctattgtatg aattgactat tacattagct atagatgatt ttaagttagt agtgggctat 240
actattaaac ttgctctaat tgttttatgc cagagctata attttatcct ttcttttgag 300
gaataaattt tatactacgg agtaacttat tccgcgatta acggttgaat atctgttatt 360
aaactaacaa tctcatcttt ttacttatac ttatatttat caatcaaaat ttaaaatttc 420
aactttaaat ttgaagttga ttttaggggt tttgatcaaa attcattttt cagtctttac 480
tttaagatca ctaaaaacac gtatatacaa gttttattaa taaattattc tttttttttt 540
ggcaaatatg ctgtttgcaa a 561

<210> 64
<211> 561
<212> DNA
<213> Rice
<400> 64
ttttgccctt aagagcaggt ataatagcaa gctataagcc agctataaac atattttaaa 60
aagataaaag aagatagaga agagcagcgg gctacagatc tgtagtcagc tgcagcacgg 120
actccaatac gtaatgtatg tatgataggt ggaaccaggt attaatagta cagtaagcaa 180
ctattgtatg aattgactat tacattagct atagatgatt ttaagttagt agtgggctat 240
actattaaac ttgctctaat tgttttatgc cagagctata attttatcct ttcttttgag 300
gaataaattt tatactacgg agtaacttat tccgcgatta acggttgaat atctgttatt 360
aaactaacaa tctcatcttt ttacttatac ttatatttat caatcaaaat ttaaaatttc 420
aactttaaat ttgaagttga ttttaggggt tttgatcaaa attcattttt cagtctttac 480
tttaagatca ctaaaaacac gtatatacaa gttttattaa taaattattc tttttttttt 540
ggcaaatatg ctgtttgcaa a 561

<210> 65
<211> 267
<212> DNA
<213> Rice
<400> 65
atagcttgga gcaagttcaa tagtatagct aactactagc tctaattcat ctataatcaa 60
tctaatagct tattcataca atagttatat actacattat taatatctgg tcccatctat 120
catacacact gagtctgtgc tatagctgac tacaaatctg tagcccgctg ctcttctctc 180
ttcatttatc ttcttaaaat atatttgcag ctggcttatg gcttatagct tgatattgag 240
agggagagga gtgagagcta gctcagc 267

<210> 66
<211> 246
<212> DNA
<213> Rice
<400> 66
atagcttgga gcaagttcaa cagtatagct aactactagc tctaattcat ctataatcaa 60
tctaatagct tattcataca atagttatat actacactat taatatctgg tcccatctat 120
tatacacaca ctgagtccgt gctatagctg actataaatc tgtagtccgc tgctcttctc 180
tctcctaatt tatctttgat attgtaactg ctattagaga gggagaggag tgagagctag 240
ctcagc 246

<210> 67
<211> 312
<212> DNA
<213> Rice
<400> 67
atagcttgga gcaagttcaa cagtatagct aactactagc tctaattcat ctataatcaa 60

tctaatagct tattcataca atagttatat actacactat taatgctcta attcatctat 120
aatcaatcta atagcttatt catacaatag ttatatacta cactattaat atctggtccc 180
atctattata cacacactga gtccgtgcta tagctgacta taaatctgta gtccgctgct 240
cttctctctc ctaatttatc tttgatattg taactgctat tagagaggga gaggagtgag 300
agctagctca gc 312

<210> 68
<211> 25
<212> DNA
<213> Artificial Sequence
<220>
<223> Description of Artificial Sequence: primer
<400> 68
attgctccct acatatgcac tcacc 25

<210> 69
<211> 17
<212> DNA
<213> Artificial Sequence
<220>
<223> Description of Artificial Sequence: primer
<400> 69
aaccgtacta agaacac 17

<210> 70
<211> 18
<212> DNA
<213> Artificial Sequence
<220>

<223> Description of Artificial Sequence: primer
<400> 70
gacaagtggt aaaggtat                                                    18

<210> 71
<211> 25
<212> DNA
<213> Artificial Sequence
<220>
<223> Description of Artificial Sequence: primer
<400> 71
gcagaaatgg agtgattgtt gcagc                                            25

<210> 72
<211> 21
<212> DNA
<213> Artificial Sequence
<220>
<223> Description of Artificial Sequence: primer
<400> 72
gagatggtac actgattact g                                                21

<210> 73
<211> 19
<212> DNA
<213> Artificial Sequence
<220>
<223> Description of Artificial Sequence: primer
<400> 73

gagttggtca atgtacaat 19

<210> 74
<211> 338
<212> DNA
<213> Rice
<400> 74
ttttccttac aagtagtctc atacatgcta atttctgtaa ggtgttgggc tggaaattaa 60
ttaattaatt aattgacttg ccaagatcca tatatatgtc ctgatattaa atcttcgttc 120
gttatgtttg gttaggctga tcaatgttat tctagagtct agagaaacac acccaggggt 180
tttccaacta gctccacaag atggtgggct agctgaccta gatttgaagt ctcactcctt 240
ataattattt tatattagat cattttctaa tattcgtgtc tttttttatt ctagagtcta 300
gatcttgtgt tcaactctcg ttaaatcatg tctctcgc 338

<210> 75
<211> 341
<212> DNA
<213> Rice
<400> 75
ttttccttac aaatagtctc atacatgcta atttctgtaa ggtgttgggc tggaaattaa 60
ttaattaatt aattaattga cttgccaaga tccatatata tgtcctgata ttaaatcttc 120
gttcgttatg tttggttagg ctgatcgatg ttattctaga gtctagagaa acatacccag 180
gggttttcca gctagctcca caagatggtg ggctagctga cctagattta agtctcactc 240
tttctaatta tttgatatta tatcattttc taatatttgc gtcttttttt attctagagt 300
ctagatcttg tgttcaactc tcgttaaatc atgtctctcg c 341

<210> 76
<211> 338
<212> DNA

<213> Rice
<400> 76
ttttccttac aagtagtctc atacatgcta atttctgtaa ggtgttgggc tggaaattaa 60
ttaattaatt aattgacttg ccaagatcca tatatatgtc ctgatattaa atcttcgttc 120
gttatgtttg gttaggctga tcaatgttat tctagagtct agagaaacac acccaggggt 180
tttccaacta gctccacaag atggtgggct agctgaccta gatttgaagt ctcactcctt 240
ataattattt tatattagat cattttctaa tattcgtgtc tttttttatt ctagagtcta 300
gatcttgtgt tcaactctcg ttaaatcatg tctctcgc 338

<210> 77
<211> 290
<212> DNA
<213> Rice
<400> 77
catgctcttg agcagcttct cgaacttctt ctttccagta ccctgaaaca cacacggatc 60
aaaattatac acatcatttg ccactgaatt gagaaatgct cagaggtctt ccggctaact 120
ccacaaggtg gtggactaga cgatctgggt tcaaagcctc accccttcta attatttgat 180
attattaggt ccttccctaa tattcgagtt ttttttttgc cactgaattt gttctgaatt 240
tttggcatat cgtgcaagtg tgtcttgtag tgtattatat tataccagaa 290

<210> 78
<211> 287
<212> DNA
<213> Rice
<400> 78
catgctcttg agcagcttct cgaacttctt ctttccagta ccctgaaaca cacacggatc 60
aaaattatac acatcatttg ccactgaatc gagaaatgct cagagctctt ccggctagct 120
ctacaaggtg gtgggctaga tgacctgggt tcaaagcctc accccttcta attatttgat 180
attaggttct tccctaatat ttgagttttt tttttgccac tgaatttgtt ctgaattttt 240

ggcatatcgt gcaagtgtgt cttgtagtgt attatattat accagaa 287

<210> 79
<211> 290
<212> DNA
<213> Rice
<400> 79
catgctcttg agcagcttct cgaacttctt ctttccagta ccctgaaaca cacacggatc 60
aaaattatac acatcatttg ccactgaatt gagaaatgct cagaggtctt ccggctaact 120
ccacaaggtg gtggactaga cgatctgggt tcaaagcctc accccttcta attatttgat 180
attattaggt ccttccctaa tattcgagtt tttttttgc cactgaattt gttctgaatt 240
tttggcatat cgtgcaagtg tgtcttgtag tgtattatat tataccagaa 290

<210> 80
<211> 461
<212> DNA
<213> Rice
<400> 80
ttcgtcaaat ataatgaagg agtaagctca acttcttgtg tccttcgaag tacagtaata 60
caaaatcacg tactaggaac actgtagaca ctatccagat cttttgtaga agaaaattta 120
acagtctgtt ggtatcactc tcatttattt ggagaaacgc ctaggggtct tccggctagc 180
tccacaaggt ggtgggctag ccggcctggg ttcgaagcct caccttttct atttatttga 240
tattaggcca ttccctaata ttcacatttt tttactctca tttattcatt atcaatatgc 300
actggtaatt gcgaaaagaa atatctgaaa tatcattttt atcaaatttc aaatgaaacc 360
aacattgact ttgagttgtt tgatcttgca gatatgtggt aactatagat ggaccagtgc 420
acagaaatca ccttatagct atcagtgaag gcacaaaaat t 461

<210> 81
<211> 461

<212> DNA
<213> Rice
<400> 81
ttcgtcaaat ataatgaagg agtaagctca acttcttgtg tccttcgaag tacagtaata 60
caaaatcacg tactaggaac actgtagaca ctatccagat cttttgtaga agaaaattta 120
acagtctgtt ggtatcactc tcatttattt ggagaaacgc ctaggggtct tccggctagc 180
tccacaaggt ggtgggctag ccggcctggg ttcgaagcct caccttttct atttatttga 240
tattaggcca ttccctaata ttcacatttt tttactctca tttattcatt atcaatatgc 300
actggtaatt gcgaaaagaa atatctgaaa tatcattttt atcaaatttc aaatgaaacc 360
aacattgact ttgagttgtt tgatcttgca gatatgtggt aactatagat ggaccagtgc 420
acagaaatca ccttatagct atcagtgaag gcacaaaaat t 461

<210> 82
<211> 461
<212> DNA
<213> Rice
<400> 82
ttcgtcaaat ataatgaagg agtaagctca acttcttgtg tccttcgaag tacagtaata 60
caaaatcacg tactaggaac actgtagaca ctatccagat cttttgtaga agaaaattta 120
acagtctgtt ggtatcactc tcatttattt ggagaaacgc ctaggggtct tccggctagc 180
tccacaaggt ggtgggctag ccggcctggg ttcgaagcct caccttttct atttatttga 240
tattaggcca ttccctaata ttcacatttt tttactctca tttattcatt atcaatatgc 300
actggtaatt gcgaaaagaa atatctgaaa tatcattttt atcaaatttc aaatgaaacc 360
aacattgact ttgagttgtt tgatcttgca gatatgtggt aactatagat ggaccagtgc 420
acagaaatca ccttatagct atcagtgaag gcacaaaaat t 461

<210> 83
<211> 224
<212> DNA

<213> Rice

<400> 83

tttttgcagg ccaaggatta atatcctgtg aattaaatta atgtgattgg ttgcgtgcat 60

cttagttatg tagaggttga gaaacgctca ggggtcttcc ggctagctcc acaaggtggt 120

gggctagccg gcctaggttc gaagccttac ccccttctat ttatttgata ctagatcctt 180

ccctaatacc tgtgttttct tttttagtta tgtagaggtt aaga 224

<210> 84

<211> 391

<212> DNA

<213> Rice

<400> 84

tttttgcagg ccaaggatta atatcctgtg cattaaatta atgtgattgg ttgtgtgcat 60

cttaattata tagaggttga gaaacgctca gaatcatccg gctagctcca caaaatggtg 120

ggctagccga ctcgtgttcg aagcttcacc cccttttatt tatttgatat taggtcattc 180

cttaatatcc gcgggacatt taattatttt gccaatttta agatgtgcca attaactatt 240

tgctactcac tgtctatgac atgtagaccc tcatgtgtct atgacatgtg ggtacagtgg 300

caaatccttt agaaccactc gtaagagtgg caaatagtta attattccga tatccgtgtt 360

ttcttttttt ttagttatgt agaggttaag a 391

<210> 85

<211> 223

<212> DNA

<213> Rice

<400> 85

tttttgcagg ccaaggatta atatcctgtg aattaaatta atgtgattgg ttgcgtgcat 60

cttagttatg tagaggttga gaaacgctca gggtcttccg gctagctcca caaggtggtg 120

ggctagccgg cctaggttcg aagccttacc cccttctatt tatttgatac tagatccttc 180

cctaatacct gtgttttctt ttttagttat gtagaggtta aga 223

<210> 86
<211> 308
<212> DNA
<213> Rice
<400> 86
gtttaaatgc caacaatgtc agccattaaa aactgaaggg agaaacgtcc tggggttttc 60
cggctagctt cacaaggtgg tgggttagac gacctgggtt cgaagcctca cccctcctaa 120
ttatttgata ttaggtcctt ccctaatact tgtgtttttc attaaaaact gaatgagtaa 180
cattttcagc ttgttggaga cagagattag cacaccacat catgtcaact atcaagaagt 240
cctatttatc atgtaccata ctattggttc ctgaaaacag tttggccatg catcactttt 300
cagtcatg 308

<210> 87
<211> 308
<212> DNA
<213> Rice
<400> 87
gtttaaatgc caacaatgtc agccattaaa aactgaaggg agaaacgtcc tggggttttc 60
cggctagctt cacaaggtag tgggctagac gatctgggtt cgaagcctca ctcctcctaa 120
ttatttgata ttaggtcctt ccctaatact tgtgattttc attaaaaact gaatgagtaa 180
cattttcagc ttgttggaga cagagattag cacaccacat catgtcaact atcaagaagt 240
cctatttatc atgtaccata ctattggttc ctgaaaacag tttggccatg catcactttt 300
cagtcatg 308

<210> 88
<211> 308
<212> DNA
<213> Rice

<400> 88

gtttaaatgc caacaatgtc agccattaaa aactgaaggg agaaacgtcc tggggttttc 60

cggctagctt cacaaggtag tgggctagac gacctgggtt cgaagcctca cccctcctaa 120

ttatttgata ttaggtcctt ccctaatact cgtgtttttc attaaaaact gaatgagtaa 180

cattttcagc ttgttggaga cagagattag cacaccacat catgtcaact atcaagaagt 240

cctatttatc atgtaccata ctattggttc ctgaaaacag tttgaccatg catcactttt 300

cagtcatg 308

<210> 89

<211> 368

<212> DNA

<213> Rice

<400> 89

tagccctgtg tgccatagat gatagaatct tcttgggcat tcccaggagt gatcctcgcg 60

ttgtctcatg gggtccagat caaccacatt cctttgaaac tgcatcctca agaaacgccc 120

aggggtcttc cagctagctc cacaaggtgg tgggctagat gacctgggtt cgaagcctca 180

cccctictaa ttatttgata ttaggtcctt ccctaatatt cgcgtctttt tttttcgaaa 240

ctgcatcctc atccatcatt cagtacagac aatcaatatg gaggttttaa atctagaaca 300

accatagatt ccatagttgg aggatttcgg agttctcaac ttatactgat caagcagtta 360

ccttgcac 368

<210> 90

<211> 367

<212> DNA

<213> Rice

<400> 90

tagccctgtg tgccatagat gatagaatct tcttgggcat tcccaggagt gatcctcgcg 60

ttgtctcatg gggtccagat caaccacatt cctttgaaac tgcatcctca agaaacgccc 120

aggcgtcttc cagctagctc cacaaggtgg tgggctagat gacctgggtt cgaagcctca 180

ccccttctaa ttatttgata ttaggtcctt ccctaatatt cgcgtctttt ttttcgaaac 240
tgcatcctca tccatcattc agtacagaca atcaatatgg aggttttaaa tctagaacaa 300
ccatagattc catagttgga ggatttcgga gttctcaact tatactgatc aagcagttac 360
cttgcac 367

<210> 91
<211> 368
<212> DNA
<213> Rice
<400> 91
tagccctgtg tgccatagat gatagaatct tcttgggcat tcccaggagt gatcctcgcg 60
ttgtctcatg gggtccagat caaccacatt cctttgaaac tgcatcctca agaaacgccc 120
aggggtcttc cagctagctc cacaaggtgg tgggctagat gacctgggtt cgaagcctca 180
ccccttctaa ttatttgata ttaggtcctt ccctaatatt cgcgtctttt tttttcgaaa 240
ctgcatcctc atccatcatt cagtacagac aatcaatatg gaggttttaa atctagaaca 300
accatagatt ccatagttgg aggatttcgg agttctcaac ttatactgat caagcagtta 360
ccttgcac 368

<210> 92
<211> 298
<212> DNA
<213> Rice
<400> 92
aagtagctat ctactgatct gtacacttct ttttgcactg gcaaactcca aagtccaaat 60
gacacagtag gattaggatt cagtagaggt cttctggcta gctccacaag gtggtaggtc 120
agttggcttg ggttcgaagt cacaccactt ctatttgttt gatattagat catttcctaa 180
aattcgcttc ttctttttct tttttttttt tggattcagt gatctatggc catgaatcat 240
cataagctag ctagcttaca cacgttacac cagcctctat ataatttagt gttggaag 298

<210> 93

<211> 298

<212> DNA

<213> Rice

<400> 93

aagtagctat ctactgatct gtacacttct ttttgcactg gcaaactcca aagtccaaat 60

gacacagtag gattaggatt cagtagaggt cttctggcta gctccacaag gtggtaggtc 120

agttggcttg ggttcgaagt cacaccactt ctatttgttt gatattagat catttcctaa 180

aattcgcttc ttctttttct tttttttttt tggattcagt gatctatggc catgaatcat 240

cataagctag ctagcttaca cacgttacac cagcctctat ataatttagt gttggaag 298

<210> 94

<211> 290

<212> DNA

<213> Rice

<400> 94

aagtagctat ctactgatct gtacacttct ttttgcactg gcaaactcca aagtccaaat 60

gacacttagg attcagtaga ggtcttctgg ctagctccac aaggtggtag gtcagtcggc 120

ttaggttcaa agtcacacca cttctatttg tttgatatta gatcatttcc taaaattcgc 180

ttcttctttt tctttttttt tttggattca gtgatctatg gccatgaatc atcataagct 240

agctagctta cacacgttac accagcctct atataattta gtgttggaag 290

<210> 95

<211> 27

<212> DNA

<213> Artificial Sequence

<220>

<223> Description of Artificial Sequence: primer

<400> 95

tcttagttat gtagaggttg agaaccg 27

<210> 96
<211> 24
<212> DNA
<213> Artificial Sequence
<220>
<223> Description of Artificial Sequence: primer
<400> 96
gatacaaccg tactaagaac actc 24

<210> 97
<211> 24
<212> DNA
<213> Artificial Sequence
<220>
<223> Description of Artificial Sequence: primer
<400> 97
ttaaatgcca acaatgtcag ccat 24

<210> 98
<211> 23
<212> DNA
<213> Artificial Sequence
<220>
<223> Description of Artificial Sequence: primer
<400> 98
gctgaaaatg ttactcattc agt 23

<210> 99

<211> 24

<212> DNA

<213> Artificial Sequence

<220>

<223> Description of Artificial Sequence: primer

<400> 99

tggtacactg attactgaag tagc                                                24

<210> 100

<211> 24

<212> DNA

<213> Artificial Sequence

<220>

<223> Description of Artificial Sequence: primer

<400> 100

cagaagacct ctactgaatc ctaa                                                24